# EUROPÄISCHE PATENTSCHRIFT

(11) **EP 2 370 576 B1**
(45) Veröffentlichungstag und Bekanntmachung des Hinweises auf die Patenterteilung: **02.03.2016**
(21) Anmeldenummer: 09799111.1
(22) Anmeldetag: 23.12.2009
(51) Int. Cl.: C12N 15/10

(54) **NUKLEINSÄUREAUFREINIGUNGSVERFAHREN**
NUCLEIC ACID PURIFICATION METHOD
PROCÉDÉ DE PURIFICATION D'ACIDES NUCLÉIQUES

(30) Priorität: 23.12.2008 DE 102008063003
(43) Veröffentlichungstag der Anmeldung: 05.10.2011
(73) Patentinhaber: Qiagen GmbH, 40724 Hilden (DE)
(72) Erfinder: FABIS, Roland, 51375 Leverkusen (DE); PETZEL, Jan, 42697 Solingen (DE); JENNRICH, Sabine, 47803 Krefeld (DE)
(74) Vertreter: Roth, Carla
(86) Internationale Anmeldenummer: PCT/EP2009/067878
(87) Internationale Veröffentlichungsnummer: WO 2010/072821

(56) Entgegenhaltungen:
- WO-A1-2007/065933
- WO-A2-2008/097342
- anonymous: "QIAGEN Purification Technologies" 15. Oktober 2005 (2005-10-15), XP002570999 Gefunden im Internet: URL:http://www1.qiagen.com/resources/info/ qiagen_purification_technologies_1.aspx> [gefunden am 2010-03-02]
- JOACHIMIAK A ET AL: "Application of spermine-Sepharose column chromatography to the separation of plant-specific transfer ribonucleic acids and aminoacyl-tRNA synthetases" JOURNAL OF CHROMATOGRAPHY, ELSEVIER SCIENCE PUBLISHERS B.V, NL, Bd. 180, Nr. 1, 28. November 1979 (1979-11-28), Seiten 157-162, XP026482690 ISSN: 0021-9673 [gefunden am 1979-11-28]
- SAKATA M ET AL: "Chromatographic Removal of Host Cell DNA from Cellular Products Using Columns Packed with Cationic Copolymer Beads", CHROMATOGRAPHIA ; AN INTERNATIONAL JOURNAL FOR RAPID COMMUNICATION IN CHROMATOGRAPHY, ELECTROPHORESIS AND ASSOCIATED TECHNIQUES, VIEWEG VERLAG, WI, vol. 62, no. 9-10, 1 November 2005 (2005-11-01), pages 465-470, XP019358534, ISSN: 1612-1112

## Beschreibung

Die vorliegende Erfindung betrifft ein Verfahren sowie einen Kit zur Aufreinigung von Nukleinsäuren aus einer nukleinsäurehaltigen Probe.

Verschiedene Verfahren zur Aufreinigung und Isolierung von Nukleinsäuren sind im Stand der Technik bekannt. Diese beinhalten den Einsatz von Phenol-Chloroform, Aussalzverfahren, den Einsatz von Ionenaustauschern sowie Silikapartikel. Ferner sind im Stand der Technik chromatographische Verfahren bekannt, bei denen die Nukleinsäuren über einen pH und/oder Salzgradienten gebunden und eluiert werden.

Ein bekanntes Verfahren zur Nukleinsäureaufreinigung ist das sog. "Charge-Switch Verfahren". Danach wird eine nukleinsäurebindende Phase bei einem ersten pH-Wert mit einer nukleinsäurehaltigen Probe in Kontakt gebracht, bei der die nukleinsäurebindende Phase eine positive Ladung aufweist. Dies begünstigt die Bindung der negativ geladenen Nukleinsäuren an die Phase. Zur Freisetzung/Elution der Nukleinsäuren wird gemäß dem Charge-Switch Prinzip ein zweiter pH-Wert eingestellt, der höher als der pKs-Wert der nukleinsäurebindenden Phase ist, um die positive Ladung zu invertieren, bzw. zu neutralisieren. Dies fördert die Ablösung der gebundenen Nukleinsäuren von der nukleinsäurebindenden Phase.

Im Stand der Technik sind sowohl lösliche Phasen (siehe bspw. EP 0 707 077) als auch feste Phasen (siehe bspw. WO 99/29703) bekannt. Zur Elution werden verschiedene Lösungen eingesetzt, so bspw. Lösungen, die einen sehr hohen pH-Wert aufweisen oder aber auch biologische Puffer, insbesondere Niedrigsalzpuffer wie bspw. Tris-Puffer, um die direkte Weiterverarbeitung der aufgereinigten Nukleinsäuren bspw. im Rahmen einer Amplifikationsreaktion oder eines Restriktionsverdaus zu ermöglichen.

Sakata et al, Chromatographia, Bd. 62, Nr. 9-10 (2005-11-01), Seiten 465-470 beschreibt ein Verfahren zur selektiven Entfernung von DNA aus zellulären Produkten unter Verwendung von Säulen, die mit kationischen Copolymer-Beads beladen sind.

Auch sind kommerzielle, auf Anionenaustauscherchromatographie basierende Verfahren bekannt, bei denen bei der Bindung und Elution variable Salzkonzentrationen und variable pH Werte eingesetzt werden (QIAGEN Anion-Exchange Resin). Zur Elution wird insbesondere ein Hochsalzpuffer eingesetzt, die eluierten Nukleinsäuren müssen daher ausgefällt werden, um sie zu entsalzen.

WO 2008/097342 offenbart ein Verfahren zur Aufreinigung Nukleinsäuren, bei dem Polykationen nicht-kovalent an einen Träger gebunden werden, um diese für die Nukleinsäurebindung herzurichten. Nach Durchführung spezieller Waschschritte, durch die sich die Polykationen von dem Träger lösen, die Nukleinsäuren von den Polykationen freigesetzt werden und die Nukleinsäuren wieder an den nackten Träger adsorbieren, kann die Nukleinsäure unter milden Bedingungen von dem nackten Träger eluiert werden. Aufgrund der speziell erforderlichen Waschschritte ist das Verfahren komplex.

WO 2007/065933 offenbart magnetische Polymerpartikel mit kovalent gebundenen Polykationen, die zur Nukleinsäuraufreinigung eingesetzt werden können.

Auch wenn die bekannten Verfahren zur Aufreinigung von Nukleinsäuren geeignet sind, besteht das Bedürfnis, die bestehenden Verfahren zu verbessern, insbesondere die Aufreinigung der Nukleinsäuren besonders schonend durchzuführen.

Der vorliegenden Erfindung liegt daher die Aufgabe zugrunde, die bestehenden Verfahren zur Aufreinigung von Nukleinsäuren zu verbessern.

Die vorliegende Erfindung löst diese Aufgabe durch ein Verfahren zu Aufreinigung von Nukleinsäuren aus einer nukleinsäurehaltigen Probe, das wenigstens die nachfolgenden Schritte aufweist:
a. Inkontaktbringen der nukleinsäurehaltigen Probe mit einer nukleinsäurebindenden Phase, welche nukleinsäurebindende Gruppen aufweist, wobei die nukleinsäurebindenden Gruppen wenigstens eine protonierbare Gruppe aufweisen, die einen pKs-Wert von 9 bis 12 aufweist, wobei die nukleinsäurebindende Phase eine feste Phase ist und die nukleinsäurebindenden Gruppen kovalent an einen Träger gebunden vorliegen und wobei die nukleinsäurebindenden Gruppen ausgewählt sind aus der Gruppe der primären, sekundären und tertiären Mono- und Polyamine und wobei der Träger und/oder die nukleinsäurebindenden Gruppen Kationenaustauscher als funktionelle Gruppen aufweisen, die beim Elutions pH-Wert die Freisetzung der Nukleinsäuren fördern;
b. Bindung der Nukleinsäuren an die nukleinsäurebindende Phase bei einem pH-Wert (Bindungs pH-Wert), der wenigstens eine pH Einheit unterhalb des pKs-Wertes wenigstens einer der protonierbaren Gruppen liegt;
c. Elution der Nukleinsäuren bei einem pH-Wert, der oberhalb des Bindungs pH Wertes liegt, jedoch wenigstens eine pH-Einheit unterhalb des pKs-Wertes wenigstens einer der protonierbaren Gruppen liegt (Elutions pH-Wert) und wobei die Elution bei einem pH-Wert von 8,2 bis 10 und bei einer Salzkonzentration von weniger als 0,25M erfolgt.

Die vorliegende Erfindung betrifft die Aufreinigung von Nukleinsäuren mittels einer nukleinsäurebindenden Phase, die entsprechend nukleinsäurebindende Gruppen aufweist. Eine solche nukleinsäurebindende Gruppe weist wenigstens eine protonierbare Gruppe auf, deren pKs-Wert bei 9 bis 12 liegt. Die Bindung der Nukleinsäuren erfolgt bei einem pH-Wert unterhalb des pKs-Wertes wenigstens einer dieser protonierbaren Gruppen. Die protonierbaren Gruppen nehmen ein Proton auf und werden dadurch positiv geladen, was dazu führt, dass die nukleinsäurebindende Phase die negativ geladenen Nukleinsäuren bindet. Die Elution erfolgt bei einem höheren pH-Wert, wodurch die positive Ladung der nukleinsäurebindenden Phase geringer wird. Der Elutions pH-Wert liegt erfindungsgemäß jedoch unterhalb des pKs-Wertes der protonierbaren Gruppen, insbesondere wenigstens eine pH-Einheit darunter, vorzugsweise wenigstens zwei pH-Einheiten darunter. Dies hat den erheblichen Vorteil, dass die Elution auch bei schonenden Bedingungen durchgeführt werden kann. Im Gegensatz zum Stand der Technik erlaubt die vorliegende Erfindung daher die Elution bei einem pH-Wert, der unterhalb der pKs-Werte der protonierbaren Gruppen liegt.

Gemäß einer Ausführungsform der vorliegenden Erfindung, erfolgt die Bindung der Nukleinsäuren bei einem pH-Wert von 3 bis 8. Diese Angabe bezieht sich auf den pH-Wert während der Bindung und damit in der Probe. Das erfindungsgemäße Verfahren ist je nach Gestaltung der festen Phase auch bei sehr schonenden Bedingungen durchführbar, so dass die Bindung der Nukleinsäuren auch bei einem pH-Wert von 4 bis 7,5; vorzugsweise bei 5 bis 7,5; besonders bevorzugt bei 5 bis 7 und ganz besonders bevorzugt bei 6,5 bis 7 und damit im nahezu neutralen Bereich durchgeführt werden kann. Aufgrund der Tatsache, dass die protonierbaren Gruppen der nukleinsäurebindenden Phase einen pKs-Wert von 9 bis 12 aufweisen, liegen diese selbst bei relativ neutralen pH-Werten ausreichend positiv geladen vor, um die effektive Anbindung der Nukleinsäuren zu erlauben. Daher kann die Bindung unter sehr schonenden Bedingungen erfolgen, was eine Schädigung der Nukleinsäuren verhindert.

Darüber hinaus hat es sich als vorteilhaft erwiesen, die Bindung bei einer niedrigen Salzkonzentration vorzunehmen. Die Salzkonzentration bei der Bindung der Nukleinsäuren an die nukleinsäurebindende Phase liegt bei weniger als 0,25 M oder sogar weniger als 0,1 M. Eine niedrige Salzkonzentration ist bevorzugt, um die Bindung der Nukleinsäuren an die feste Phase zu optimieren. Zu hohe lonenkonzentrationen beeinflussen die ionischen Wechselwirkungen von Nukleinsäure und der nukleinsäurebindenden Phase negativ. Es hat sich herausgestellt, dass der Bindungspuffer auch gewisse Mengen organische Substanzen wie bspw. Kohlenhydrate, Alkohole wie bspw. Ethanol, Methanol oder Aceton und Acetonitrid enthalten kann. Diese beeinträchtigen die Bindung nicht.

Ein weiterer wesentlicher Schritt des vorliegenden Verfahrens ist die Elution der Nukleinsäuren. Wie dargelegt, erfolgt die Nukleinsäurefreisetzung bei einem pH-Wert, der oberhalb des Bindungs pH-Wertes liegt. Dies hat zur Folge, dass die protonierbaren Gruppen bei der Elution weniger positiv geladen vorliegen, wodurch die Freisetzung der Nukleinsäuren begünstigt wird. Darüber hinaus liegt der pH-Wert bei der Elution wenigstens eine pH-Einheit unterhalb des pKs-Wertes wenigstens einer der protonierbaren Gruppen der nukleinsäurebindenden Phase. Dies hat wie oben dargelegt zur Folge, dass die Elution unter besonders schonenden Bedingungen durchgeführt werden kann.

Vorzugsweise erfolgt die Elution je nach eingesetzter nukleinsäurebindender Gruppe bzw. nukleinsäurebindenden Phase bei einem pH-Wert von 8,2 bis 9 bzw. 8,2 bis 8,8. Bei diesen niedrigen pH-Werten werden besonders vorteilhafte Ergebnisse erzielt, da die Nukleinsäuren besonders schonend freigesetzt werden. Weitere Maßnahmen, die eine besonders effiziente Freisetzung der Nukleinsäuren bei einem niedrigen pH-Wert ermöglichen, werden nachfolgend beschrieben.

Um die direkte Weiterverarbeitung der isolierten Nukleinsäuren im Elutionspuffer zu ermöglichen, weist dieser eine niedrige Salzkonzentration auf. Die Salzkonzentration liegt daher bei weniger als 0,25 M, bei weniger als 0,1 M, besonders bevorzugt bei weniger als 50 mM, weniger als 25 mM oder sogar weniger als 10 mM. Geeignete Salze können Chloride der Alkali und Erdalkalimetalle oder Ammonium, andere Salze von Mineralsäuren, Acetate, Borate sowie Verbindungen wie Tris, Bis-Tris sowie organische Puffer, wie bspw. MES, CHAPS, HEPES und ähnliche darstellen. Geeignete Substanzen zur Elution sind darüber hinaus im Stand der Technik bekannt.

Um die Aufreinigung zu begünstigen, wird nach der Bindung und vor der Elution der Nukleinsäuren vorzugsweise wenigstens ein Waschschritt durchgeführt. Zum Waschen sind wässrige Lösungen mit niedrigen Salzkonzentrationen und aber auch Wasser bevorzugt. Wenn Salze in den Waschpuffern enthalten sind, so bevorzugt in einer Konzentration von weniger als 400 mM, besonders bevorzugt von weniger als 200 mM, 100 mM, 50 mM und/oder sogar weniger als 25 mM. Organische Bestandteile können in dem Waschpuffer enthalten sein, so bspw. Alkohole, Polyole, Polyethylenglykole, Aceton, Acetonitrid oder Kohlenhydrate. Die Waschpuffer können jedoch ohne störende Mengen der entsprechenden organischen Bestandteile vorliegen, so dass nachfolgende Anwendungen, wie bspw. enzymatische Verarbeitungen, Amplifikationsreaktionen und dergleichen ("downstream" Applikationen) nicht behindert werden.

Die erfindungsgemäß einzusetzende nukleinsäurebindende Phase ist fest.

Die nukleinsäurebindende Phase ist eine feste Phase. Zur Herstellung können die protonierbaren Gruppen an ein festes Trägermaterial gebunden werden. Einzelheiten werden nachfolgend noch im Detail beschrieben. Der Einsatz einer festen Phase erleichtert die Abtrennung der gebundenen Nukleinsäuren von der Probe. Gemäß einer Ausführungsform erfolgt daher nach der Bindung der Nukleinsäuren eine Abtrennung der festen Phase.

Die protonierbaren Gruppen sind bzw. weisen gemäß einer Ausführungsform Ionenaustauscher auf; vorzugsweise Anionenaustauscher. Bevorzugte protonierbare Gruppen, die sich für die Bindung von Nukleinsäuren bewährt haben, sind Aminogruppen, bevorzugt sind primäre und sekundäre Aminogruppen. Vorzugsweise weisen die Aminogruppen einen pKs-Wert von 9 bis 12, vorzugsweise 10 bis 12 auf. Die nukleinsäurebindende Gruppe weist vorzugsweise 1 bis 10, besonders bevorzugt 2 bis 8 und insbesondere 2 bis 6 Aminogruppen auf. Bevorzugte nukleinsäurebindende Gruppen sind bspw. primäre, sekundäre und tertiäre Mono- und Polyamine. Diese können substituiert oder unsubstituiert sein. Beispiele sind insbesondere Amine der Formeln

R₁R₂R₃N,

R₁R₂N(CH₂)nNR₃R₄

R₁R₂N(CH₂)ₙNR₃(CH₂)ₘNR₄R₅

R₁R₂N(CH₂)ₙNR₃(CH₂)ₘNR₄(CH₂)ₒNR₅R₆

R₁R₂N(CH₂)ₙNR₃(CH₂)ₘNR₄(CH₂)ₒNR₅(CH₂)pNR₆R₇

R₁R₂N(CH₂)ₙNR₃(CH₂)ₘNR₄(CH₂)ₒNR₅(CH₂)ₚNR₆(CH₂)_{q}NR₇R₈

R₁R₂N(CH₂)ₙNR₃(CH₂)ₘNR₄(CH₂)ₒNR₅(CH₂)ₚNR₆(CH₂)_{q}NR₇(CH₂)ᵣNR₈R₉

R₁R₂N(CH₂)ₙNR₃(CH₂)ₘNR₄(CH₂)ₒNR₅(CH₂)pNR₆(CH₂)qNR₇(CH₂)ᵣNR₈(CH₂)ₛNR₉R₁₀

wobei
n, m, o, p, q, r und s unabhängig voneinander 2 bis 8 sind;
R₁, R₂, R₃, R₄, R₅, R₆, R₇, R₈, R₉ und R₁₀ gleich oder verschieden sind und ausgewählt sind aus der Gruppe H, Alkyl (verzweigt oder unverzweigt) und Aryl.

Besonders bevorzugt sind N-Propyl-1,3-propandiamin und Pentaethylenhexamin und ganz besonders bevorzugt Spermin und Spermidin als nukleinsäurebindende Gruppe.

Darüber hinaus können auch ringförmige Amine, aromatische Amine oder aminofunktionalisierte Heterozyklen eingesetzt werden. Die Amine können Substituenten tragen, bspw. Alkyl-, Alkenyl-, Alkinyl- oder aromatische Substituenten, darüber hinaus können die Kohlenwasserstoffketten auch zu einem Ring geschlossen sein. Die Kohlenwasserstoffketten können auch Heteroatome, wie Sauerstoff, Stickstoff, Schwefel oder Silizium, oder Verzweigungen aufweisen.

Weitere geeignete nukleinsäurebindende Gruppen sind Polyoxyalkylenamine mit einer, zwei oder drei Aminogruppen. Diese sind beispielsweise erhältlich unter dem Namen "Jeffamine" Polyoxylalkylenamine. Jeffamine enthalten primäre Aminogruppen, die an den Terminus des Polyetherbackbones gebunden sind. Das Polyetherbackbone kann auf Propylenoxid, Ethylenoxid oder Mischungen daraus basieren; auch ist der Einsatz anderer Backbonesegmente denkbar.

Wie ausgeführt weisen die Aminogruppen der Amine pKs-Werte von 9 bis 12, bevorzugt von 10 bis 12 auf.

Auch Mischungen der entsprechenden nukleinsäurebindenden Gruppen können erfindungsgemäß eingesetzt bzw. auf einen Träger aufgebracht werden.

Die nukleinsäurebindenden Gruppen wie bspw. die Amine werden kovalent an den Träger gebunden. Vorzugsweise werden sie so verknüpft, dass pro angebundener Gruppe ein (bspw. N-Propyl-1,3-propandiamin) bis zehn, bevorzugt zwei bis acht, besonders bevorzugt zwei bis sechs Aminogruppen als protonierbare Gruppe vorhanden sind.

Vorzugsweise stehen die Aminogruppen der nukleinsäurebindenden Gruppen nicht in Konjugation mit einer die Elektronendichte verringernden Gruppe wie bspw. einer Carboxylgruppe, einer Carbonylgruppe, einer Gruppe mit C-C-Doppelbindungen oder einer ß-Hydroxyethylgruppe, so dass ihr pKs-Wert zwischen 9 und 12 liegt. Eine Konjugation mit einer Elektronendichte verringernden Gruppe gilt dann als vorhanden, wenn eine Aminofunktion und die entsprechende, die Elektronendichte verringernde Gruppe über nur drei, zwei oder weniger Kohlenstoffatome verbunden sind.

Die nukleinsäurebindenden Gruppen sind an einen Träger gebunden, so dass eine feste nukleinsäurebindende Phase für die Nukleinsäureaufreinigung eingesetzt wird. Als Träger für die nukleinsäurebindenden Gruppen kommen bspw. organische Polymere wie Polystyrol und seine Derivate, Polyacrylate und -methacrylate, und ihre Derivate oder Polyurethane, Nylon, Polyethylen, Polypropylen, Polybutylen, und Copolymere aus diesen Materialien infrage. Darüber hinaus können diese nukleinsäurebindenden Gruppen auch mit Polysacchariden, insbesondere Hydrogelen wie Agarose, Cellulose, Dextran, Sephadex, Sephacryl, Chitosan verknüpft sein. Des weiteren können die nukleinsäurebindenden Gruppen auch an anorganische Träger wie bspw. Kieselgel, Glas oder weitere Metall- und Halbmetalloxide, Silika, Boroxid oder Metalloberflächen, wie z.B. Gold, angebunden werden. Magnetische Partikel sind besonders vorteilhaft in der Handhabung. Die nukleinsäurebindenden Gruppen können direkt oder aber über Abstandshalter, sogenannte "Spacer", an diese Träger gebunden sein. Sie können auch Teil eines größeren Moleküls sein. Beispiele für Spacer sind Kohlenwasserstoffketten, Poylethylen- oder Polypropylenglykole, sowie funktionalisierte Silane. Diese Spacer können verzweigt oder unverzweigt vorliegen.

Als chemische Funktionalitäten für die Anbindung der nukleinsäurebindenden Gruppen können Säureamide oder - anhydride, Epoxide, Tosylgruppen, Formylgruppen, Sulfonylchloride, Maleinimide oder mit Carbodiimidchemie aktivierte Carboxylatgruppen eingesetzt werden. Die nukleinsäurebindenden Gruppen können auch über Thiole an z.B. Goldoberflächen angebunden sein. Bevorzugt ist die Anbindung der nukleinsäurebindenden Gruppen an carboxylierte Oberflächen.

Weitere Ausführungsformen der Trägermaterialien umfassen nichtmagnetische und magnetische Partikel, Säulenmaterialien, Membranen, sowie Oberflächenbeschichtungen. Ferner seien funktionalisierte Träger wie Röhrchen, Membrane, Vliese, Papier, Reaktionsgefäße wie PCR-Gefäße, "Eppendorf-Tubes", Multiplates, Chips und Mikroarrays genannt.

Überraschenderweise wurde gefunden, dass die anwendbaren pH-Werte und Salzkonzentrationen im Bindungs- und Elutionspuffer mit der Anzahl der vorhandenen protonierbaren Gruppen, insbesondere Aminogruppen pro nukleinsäurebindender Gruppe korrelieren. So bindet bei einer Salzkonzentration von ca. 50 mM die Nukleinsäure bereits bei einem pH-Wert von 6 an mit Spermin beschichtete Oberflächen, während für eine Anwendung einer mit N-Propyl-1,3-Proprandiamin beschichteten Oberfläche ein niedrigerer pH-Wert von 5,5, vorzugsweise sogar 5 bevorzugt ist. Die Elution von N-Propyl-1,3-Propandiamin Oberflächen gelingt bereits bei einem pH-Wert von 7,5, während bei mit Spermin beschichteten Oberflächen ein pH-Wert von ca. 8,5 bei einer Salzkonzentration von 50 mM benötigt wird. Durch Modifikation des Trägers (siehe unten) kann der pH-Wert zur Elution jedoch auch noch gesenkt werden.

Besonders entscheidend für den Wirkungsgrad der Nukleinsäureaufreinigung ist eine effiziente Elution und damit Ablösung der gebundenen Nukleinsäuren von der nukleinsäurebindenden Phase. Hier wurde überraschend festgestellt, dass nicht nur die pKs-Werte der protonierbaren Gruppen der nukleinsäurebindenden Gruppen entscheidend sind. Auch die Struktur der nukleinsäurebindenden Phase und die Anwesenheit anderer funktioneller Gruppen tragen dazu bei, die Elution bei pH-Werten im neutralen bzw. schwach alkalischen Bereich zu begünstigen und zu verbessern.

Die nukleinsäurebindende Phase trägt zusätzlich funktionelle Gruppen, die beim Elutions pH-Wert die Elution der Nukleinsäuren fördern, bspw. indem sie einen abstoßenden Effekt ausüben. Vorzugsweise liegen diese funktionellen Gruppen daher bei der Elution negativ geladen vor. Die pKs-Werte dieser Gruppen können bspw. in einem Bereich von 0 bis 7, vorzugsweise 1 bis 5 liegen. Geeignet sind bspw. Ionenaustauscher, insbesondere Kationenaustauscher, bevorzugt saure Gruppen wie bspw. Carboxylgruppen. Weitere geeignete Gruppen sind Betaine, Sulfonate, Phosphonate und Phosphate. Bspw. kann der feste Träger mit Carboxylgruppen funktionalisiert sein, um eine Anbindung der nukleinsäurebindenden Gruppen zu ermöglichen. Bei der Anbindung der nukleinsäurebindenden Gruppen kann die Konzentration selbiger so gewählt sein, dass einige der Carboxylgruppen frei vorliegen und daher nicht mit den nukleinsäurebindenden Gruppen funktionalisiert werden. Diese behindern bei niedrigen pH-Werten die Bindung der Nukleinsäuren nicht. Bei höheren pH-Werten liegen diese jedoch vorzugsweise negativ geladen vor und begünstigen dadurch die Ablösung der Nukleinsäuren von den nukleinsäurebindenden Gruppen. Diese Wechselwirkung kann ferner durch die Auswahl der Länge bzw. des Abstandes zwischen den protonierbaren Gruppen der nukleinsäurebindenden Gruppen und den negativ ionisierbaren Gruppen, wie bspw. den Carboxylgruppen begünstigt werden. Dadurch wird in vorteilhafter Weise die Elution bei niedrigen pH-Werten begünstigt, so dass die Ausbeute steigt. Die Auswahl, Stärke und Länge der funktionellen Gruppen, die bei dem Elutions pH-Wert einen abstoßenden Effekt auf die Nukleinsäuren ausüben, variiert je nach ausgewählter nukleinsäurebindender Gruppe, so insbesondere der Anzahl der protonierbaren Gruppen pro nukleinsäurebindender Gruppe und deren Abstand zu den die Elution fördernden funktionellen Gruppen.

Ferner hat sich gezeigt, dass die Elutionseffizienz auch gesteigert werden kann, wenn die nukleinsäurebindenden/protonierbaren Gruppen beabstandet zueinander auf dem Trägermaterial angeordnet bzw. verdünnt werden. Um eine solche Anordnung der nukleinsäurebindenden Gruppen zu erreichen, kann gemäß einer Ausführungsform der Träger nur mit geringen Mengen an nukleinsäurebindenden Gruppen beschichtet werden. Vorzugsweise erfolgt die Funktionalisierung mit nukleinsäurebindenden Gruppen daher im Unterschuss. Dadurch wird quasi eine Verdünnung der nukleinsäurebindenden Gruppen auf dem Träger erreicht, so dass weniger nukleinsäurebindende Gruppen zur Verfügung stehen. Dies begünstigt die Elution, da eine weniger starke Bindung der Nukleinsäuren erfolgt und diese dadurch wieder leichter von der nukleinsäurebindenden Phase abgelöst werden können. Bspw. können nur ≤ 50%, ≤ 25%, ≤ 15%, ≤ 10% oder nur ≤ 5% der funktionellen Gruppen auf dem Trägermaterial mit nukleinsäurebindenden Gruppen funktionalisiert werden. Sofern das Trägermaterial keine geeigneten funktionellen Gruppen zur Anbindung der nukleinsäurebindenden Gruppen aufweist, kann zunächst eine Funktionalisierung des Trägermaterials erfolgen, um dieses mit geeigneten funktionellen Gruppen auszustatten (siehe oben). Hierbei kann das entsprechende Profil der Beschichtung des Trägermaterials mit nukleinsäurebindenden Gruppen im Unterschuss auch dadurch erzielt werden, dass das Trägermaterial entsprechend mit weniger funktionellen Gruppen zur Anbindung der nukleinsäurebindenden Gruppen ausgestattet wird.

Gemäß einer weiteren Ausführungsform erfolgt eine Beschichtung des Trägers mit einer Mischung aus nukleinsäurebindenden Gruppen und sog. Verdünnungsgruppen. Der Begriff "Verdünnungsgruppen" wird hier dazu verwendet, um seine Funktion im Verhältnis zu den nukleinsäurebindenden Gruppen darzustellen. Ihre Funktion ist es, die Menge an nukleinsäurebindenden Gruppen auf dem Träger einzustellen und dadurch auch die Stärke der Bindung der Nukleinsäuren zu beeinflussen. Je höher der Anteil an Verdünnungsgruppen ist, desto weniger nukleinsäurebindende Gruppen werden auf den Träger aufgebracht und desto geringer ist die Stärke der Bindung an die Nukleinsäuren. Die Verdünnungsgruppen können negativ, positiv oder neutral geladen sein bzw. ionisierbare Gruppen aufweisen. Die Verdünnungsgruppen können daher auch gleichzeitig funktionelle Gruppen aufweisen, die die Elution fördern (siehe oben). Der Anteil an nukleinsäurebindenden Gruppen im Verhältnis zu den Verdünnungsgruppen kann bspw. ≤ 50%, ≤ 25%, ≤ 15%, ≤ 10% oder nur ≤ 5% betragen. Geeignete Verdünnungsgruppen sind bspw. Amine, Dimethylamin, Diethylamin und Ammoniak. Gemäß einer bevorzugten Ausführungsform wird eine Mischung aus gängigen (im Stand der Technik bekannten) Monoaminen und erfindungsgemäßen Polyaminen auf den Träger aufgebracht. Die Polyamine dienen in dieser Konstellation der Anbindung der Nukleinsäuren, die Monoamine fungieren primär als Verdünnungsgruppen. Ein Beispiel für eine geeignete Verdünnungsgruppe wäre Ethanolamin.

Durch Wahl/Kombination der beschriebenen Parameter, insbesondere der die Elution fördernden funktionellen Gruppen, der Verdünnungsgruppen, sowie der Verdünnung bzw. Mischung mit nukleinsäurebindenden Gruppen kann der pH-Wert der nukleinsäurebindenden Phase in Bezug auf die Elutionsbedingungen optimal eingestellt werden. Entsprechend kann das Elutionsprofil der nukleinsäurebindenden Phase, insbesondere der Elutions pH-Wert gesteuert bzw. eingestellt werden.

Nukleinsäuren, die mit den erfindungsgemäßen Systemen aufgereinigt werden können, können in Körperflüssigkeiten wie Blut, Urin, Stuhl, Speichel, Sputum, oder sonstigen Körperflüssigkeiten, in biologischen Quellen wie Gewebe, Zellen, insbesondere Tierzellen, Humanzellen, Pflanzenzellen, Bakterienzellen und dergleichen, Organen wie Leber, Niere oder Lunge etc. vorliegen. Dazu kann die Nukleinsäure aus Trägermaterialien wie Swabs, PapSmears, sowie stabilisierenden Medien wie PreServCyt oder Surepath, oder auch aus weiteren Flüssigkeiten, wie z.B. Säften, wässrigen Proben oder allgemein Lebensmitteln gewonnen werden. Darüber hinaus können die Nukleinsäuren aus Pflanzenmaterial, bakteriellen Lysaten, in Paraffin eingebettetem Gewebe, wässrigen Lösungen oder Gelen gewonnen werden.

Darüber hinaus betrifft die vorliegende Erfindung die Verwendung einer nukleinsäurebindenden Phase wie oben beschrieben zur Aufreinigung von DNA. Die nukleinsäurebindende Phase, die erfindungsgemäß zum Einsatz kommt, weist nukleinsäurebindende Gruppen mit wenigstens einer protonierbaren Gruppe auf, die einen pKs-Wert von 9 bis 12 aufweist, wobei die nukleinsäurebindende Phase eine feste Phase ist und die nukleinsäurebindenden Gruppen kovalent an einen Träger gebunden vorliegen und wobei die nukleinsäurebindenden Gruppen ausgewählt sind aus der Gruppe der primären, sekundären und tertiären Mono- und Polyamine und wobei die nukleinsäurebindende Phase zusätzlich Kationenaustauscher als funktionelle Gruppen aufweist, die bei dem Elutions pH-Wert die Freisetzung/Elution der Nukleinsäuren begünstigen. Bei der erfindungsgemäßen Verwendung wird ein Elutions pH-Wert eingestellt, der oberhalb des Bindungs pH-Wertes liegt, jedoch wenigstens eine pH Einheit unterhalb des pKs-Wertes wenigstens einer der protonierbaren Gruppen liegt und die Elution erfolgt bei einem pH-Wert von 8,2 bis 10 und bei einer Salzkonzentration von weniger als 0,25M. Bevorzugte Ausgestaltungen sind oben im Detail beschrieben (siehe obige Offenbarung) und insbesondere durch ein oder mehrere der folgenden Merkmale gekennzeichnet:
a) als Kationenaustauscher liegen Carboxylgruppen vor; und/oder
b) die nukleinsäurebindende Phase nach Merkmal b) oder c) weist einen Träger auf, der ausgewählt ist aus der Gruppe bestehend aus organischen Polymeren wie Polystyrol und seinen Derivaten, Polyacrylaten und -methacrylaten und ihren Derivaten, Polyurethanen, Nylon, Polyethylen, Polypropylen, Polybutylen und Copolymeren aus diesen Materialien, Polysacchariden und Hydrogelen wie Agarose, Cellulose, Dextran, Sephadex, Sephacryl, Chitosan, anorganischen Trägern, insbesondere Kieselgelen, Silikapartiken, Glas oder weiteren Metall- und Halbmetalloxiden, Boroxid, Trägern mit Metalloberflächen, wie z.B. Gold, und magnetischen Partikeln; und/oder
c) die nukleinsäurebindenden Gruppen der nukleinsäurebindenden Phase weisen primäre oder sekundäre Amine auf; und/oder
d) die nukleinsäurebindenden Gruppen nach Merkmal c) sind insbesondere Spermin und/oder Spermidin.

Ferner wird mit der Erfindung ein Kit zur Aufreinigung von DNA zur Verfügung gestellt, welcher dadurch gekennzeichnet ist, dass es
a) eine erfindungsgemäße nukleinsäurebindende Phase aufweist, welche nukleinsäurebindende Gruppen mit wenigstens einer protonierbaren Gruppe aufweist, die einen pKs-Wert von 9 bis 12 aufweist und die nukleinsäurebindenden Gruppen ausgewählt sind aus N-Propyl-1,3-propandiamin, Pentaethylenhexamin, Spermin und Spermidin und wobei die nukleinsäurebindende Phase eine feste Phase ist und die nukleinsäurebindenden Gruppen kovalent an einen Träger gebunden vorliegen und wobei die nukleinsäurebindende Phase zusätzlich Kationenaustauscher als funktionelle Gruppen aufweist, die bei dem Elutions pH-Wert die Freisetzung/Elution der Nukleinsäuren begünstigen;
b) einen Bindungspuffer mit einem pH-Wert, der wenigstens eine pH-Einheit unterhalb des pKs-Wertes wenigstens einer der protonierbaren Gruppen der nukleinsäurebindenden Phase liegt und/oder einen Bindungspuffer, der die Einstellung eines solchen pH-Wertes in der Probe ermöglicht, aufweist;
c) einen Elutionspuffer mit einem pH-Wert, der wenigstens eine pH-Einheit unterhalb des pKs-Wertes wenigstens einer der protonierbaren Gruppen der nukleinsäurebindenden Phase aber oberhalb des pH-Wertes des Bindungspuffers liegt und der einen pH-Wert von 8,2 bis 10 und eine Salzkonzentration von weniger als 0,25M aufweist.

Einzelheiten zur nukleinsäurebindenden Phase sowie den Elutionsbedingungen sind im Detail oben beschrieben und gelten auch im Zusammenhang mit dem erfindungsgemäßen Kit und kennzeichnen die darin verwendeten Bestandteile/Puffer. Wir verweisen auf die obige Offenbarung. Darüber hinaus kann das Kit weitere übliche Bestandteile enthalten, so bspw. Lyse-, Wasch - und/oder Neutralisierungsreagenzien bzw. Puffer.

Der Bindungspuffer kann vorzugsweise wenigstens eines der nachfolgenden Merkmale aufweisen:
i. einen pH-Wert von 3 bis 8; und/oder
ii. einen pH-Wert von 4 bis 7,5; und/oder
iii. einen pH-Wert von 4,5 bis 7; und/oder
iv. einen pH-Wert von 5,5 bis 7; und/oder
v. einen pH-Wert von 6,5 bis 7; und/oder
vi. eine Salzkonzentration von weniger als 1 M, weniger als 0,5M, weniger als 0,25M oder weniger als 0,1 M.

Die Vorteile der entsprechenden Merkmale wurden oben bereits im Zusammenhang mit dem Verfahren erläutert, wir verweisen auf die obige Offenbarung.

Der erfindungsgemäße Elutionspuffer kann wenigstens eines der nachfolgenden Merkmale aufweisen:
i. einen pH-Wert von 8,2 bis 9; und/oder
ii. einen pH-Wert von 8,2 bis 8,8; und/oder
iii. eine Salzkonzentration von weniger als 0,1 M, weniger als 25mM, weniger als 15mM oder weniger als 10mM; und/oder
iv. er ist ausgewählt aus der Gruppe bestehend aus Wasser, biologische Puffer, organische Puffer, insbesondere Tris, Tris-Bis, MES, CHAPS und HEPES.

Einzelheiten und Vorteile der entsprechenden Merkmale wurden bereits oben im Zusammenhang mit dem erfindungsgemäßen Verfahren im Detail erläutert. Wir verweisen auf die obige Offenbarung.

Die entsprechenden Kits können insbesondere im Rahmen des erfindungsgemäßen Verfahrens zur Anwendung kommen. Die vorliegenden Verfahren, Kits sowie nukleinsäurebindenden festen Phasen können insbesondere im Bereich der Molekularbiologie, der molekularen Diagnostik, in der Forensik, in der Lebensmittelanalytik sowie im Applied Testing eingesetzt werden.

Die eluierten Nukleinsäuren können vorzugsweise direkt weiterverarbeitet werden, so bspw. im Rahmen einer PCR, RT-PCR, einem Restriktionsverdau oder einer Transkription zum Einsatz kommen. Eine weitere Aufreinigung ist nicht erforderlich, sofern die Elutionspuffer wie oben beschrieben ausgestaltet sind und vorzugsweise eine niedrige Salzkonzentration aufweisen.

Als Nukleinsäuren kommen für eine Aufreinigung DNA in Frage, insbesondere genomische DNA, Plasmid-DNA, sowie PCR Fragmente, cDNA, sowie Oligonukleotide und modifizierte Nukleinsäuren wie bspw. PMA oder LMA. Es können auch virale oder bakterielle DNA oder Nukleinsäuren aus menschlichen, tierischen oder pflanzlichen Quellen aufgereinigt werden. Des weiteren kommen für eine erfindungsgemäße Aufreinigung auch DNA/RNA Hybride in Frage.

Die vorliegende Erfindung soll nachfolgend anhand von einigen Beispielen erläutert werden. Diese sind nicht beschränkend, stellen jedoch bevorzugte Ausführungsformen der vorliegenden Erfindung dar. Darüber hinaus werden sämtliche der hierin genannten Referenzen zum Gegenstand der Offenbarung gemacht.

### BEISPIELE

Im Rahmen der Experimente wurden als Modellsysteme für Nukleinsäuren Plasmid-DNA aus pUC21, ungeschnitten, RNA, sowie genomische DNA eingesetzt. Darüber hinaus wurde die Aufreinigung von Nukleinsäurefragmenten unterschiedlicher Größe mit von Restriktionsenzymen in Fragmente geschnittener Plasmid-DNA demonstriert.

Die Durchführung (im experimentellen Teil) erfolgte gemäß der nachstehenden Herstellungsvorschriften A) bis I):

### A) Umsetzung von magnetischen Polymeren mit Aminen

### Materialien

**Magnetisches Polymer:** Sera-Mag Double Speed Magnetic Carboxylate-Modified Microparticles (dsMGCM) Katalog No. 65152105050250, 5% ige wässrige Suspension, bzw. Magnetic Carboxylate-Modified (MG-CM), Katalog-Nr. 2415-2105, 5%ige wässrige Suspension, Seradyn Inc. Indianapolis, USA.

**Amine:** Spermin (Fluka, 85590), Spermidin (Fluka, 85561), Propyl-1,3-propandiamin (Aldrich, Katalog-Nr. 308153), Pentaethylenhexamin (Aldrich, Katalog-Nr. 292753) Poly(allylamin-hydrochloride), Mw 15.000 (Aldrich, Katalog-Nr. 283215)

Man resuspendiert 500 mg der magnetischen Partikel in 10ml 50 mM MES Puffer, pH 6,1, und setzt dann 11,5 ml einer 50 mg/ml Lösung von N-Hydroxysuccinimid zu. Man mischt mit einem Minishaker, setzt dann 10 ml einer 52 µmol/l Lösung von 1-Ethyl-3-(3-dimethylaminopropyl) carbodiimid (EDC) zu und vortext erneut. Anschließend lässt man 30 Minuten am Überkopfschüttler reagieren und trennt dann die Überstände ab. Man resuspendiert in 50 ml, 50 mM MES Puffer, pH 6,1, und verteilt dann in 10 ml Aliquots. Die Suspension wird magnetisch separiert und die Überstände verworfen. Dann nimmt man in 1 ml 50 mM MES Puffer, pH 6,1 auf und setzt je 2 ml des Amins in einer Konzentration von 100 mg/ml in 50 mM MES und einem pH-Wert von 8,5 zu, vortext gründlich, übt 10 Minuten lang Ultraschall aus und lässt eine Stunde am Überkopfschüttler reagieren. Anschließend wäscht man zweimal mit je 10 ml 50 mM MES Puffer, pH 6,1, separiert magnetisch und verwirft die Überstände. Die Partikel werden dann in je 2 ml MES-Puffer mit pH-Werten von 4,5 bis 7,0 resuspendiert.

### B) Aufreinigung von Plasmid pUC21 mit N-Propyl-1,3-propandiamin funktionalisierten magnetischen Polymeren (AX027)

Es werden 2 mg der magnetischen Partikel in 50 mM MES-Puffer, pH 5,0 bzw. 5,5, eingesetzt und jeweils mit 50 µl 50 mM MES-Puffer, pH 5,0 bzw. 5,5, versetzt. Dann setzt man 10 µg Plasmid pUC21 in 10 µl Puffer "EB" (QIAGEN, Katalog-Nr. 19068) zu und mischt durch kurzes Schütteln. Anschließend inkubiert man das Reaktionsgemisch 5 Minuten lang am Überkopfschüttler oder Eppendorf-Schüttler. Das Probengemisch wird magnetisch separiert und die Überstände entnommen und der Gehalt von DNA photometrisch bestimmt. Anschließend wäscht man die Rückstände zweimal mit je 100 µl Millipore-Wasser, separiert magnetisch und verwirft die Überstände. Dann eluiert man zweimal durch Zugabe von je 50µl 50 mM Tris-Puffer, pH 8,5 mit NaCl-Konzentrationen von 50 mM, 100 mM, 200 mM und 400 mM, trennt mittels magnetischer Separation ab und untersucht die Eluate photometrisch auf deren DNA-Gehalt.

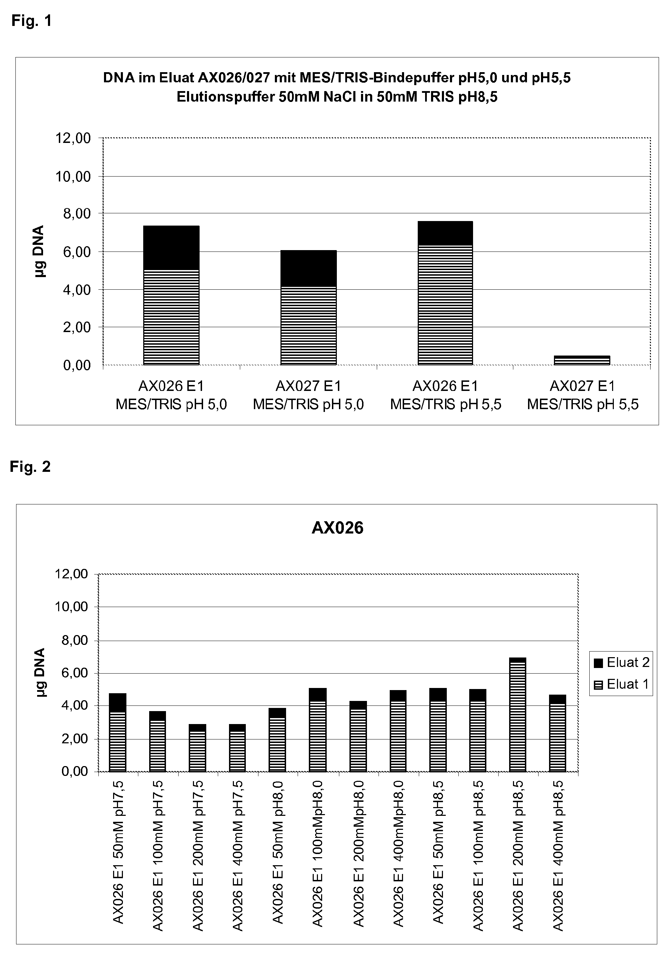

Die Ergebnisse für NaCl-Konzentrationen von 50 mM, auch im Vergleich zu AX 026 und AX 027 sind in **FIG. 1** dargestellt.

### C) Aufreinigung von Plasmid pUC21 mit Spermidin funktionalisierten magnetischen Polymeren (AX 026)

Es werden 2 mg der magnetischen Partikel in 50 mM MES-Puffer, pH 6,2, eingesetzt und mit 50 µl 50 mM Tris-Puffer, pH 6,2, versetzt. Dann setzt man 10 µg Plasmid pUC21 in 10 µl Puffer "EB" (QIAGEN, Katalog-Nr. 19068) zu und mischt durch kurzes Schütteln. Anschließend inkubiert man das Reaktionsgemisch 5 Minuten lang am Überkopfschüttler oder Eppendorf-Schüttler. Das Probengemisch wird magnetisch separiert und die Überstände entnommen und der Gehalt von DNA photometrisch bestimmt. Anschließend wäscht man die Rückstände zweimal mit je 100 µl Millipore-Wasser, separiert magnetisch und verwirft die Überstände. Dann eluiert man zweimal durch Zugabe von je 50µl 50 mM Tris-Puffer, pH 7,5, 8,0 und 8,5 mit NaCl-Konzentrationen von jeweils 50 mM, 100 mM, 200 mM und 400 mM, trennt mittels magnetischer Separation ab und untersucht die Eluate photometrisch auf deren DNA-Gehalt.

Die Ergebnisse sind in FIG. 2 dargestellt.

### D) Aufreinigung von Plasmid pUC21 mit Spermin funktionalisierten magnetischen Polymeren (AX 025)

Es werden 2 mg der magnetischen Partikel in 50 mM MES-Puffer, pH 6,1, eingesetzt und mit 50µl 50 mM Tris-Puffer, pH 7,0, versetzt. Dann setzt man 10 µl Plasmid pUC21 in 10µl Puffer "EB" (QIAGEN, Katalog-Nr. 19068) zu und mischt durch kurzes Schütteln. Anschließend inkubiert man das Reaktionsgemisch 5 Minuten lang am Überkopfschüttler oder Eppendorf-Schüttler. Das Probengemisch wird magnetisch separiert und die Überstände entnommen und der Gehalt von DNA photometrisch bestimmt. Anschließend wäscht man die Rückstände zweimal mit je 100µl Millipore-Wasser, separiert magnetisch und verwirft die Überstände. Dann eluiert man zweimal durch Zugabe von je 50µl 50 mM Tris-Puffer, pH 7,5, 8,0 und 8,5 mit NaCl-Konzentrationen von jeweils 50 mM, 100 mM, 200 mM und 400 mM, trennt mittels magnetischer Separation ab und untersucht die Eluate photometrisch auf deren DNA-Gehalt.

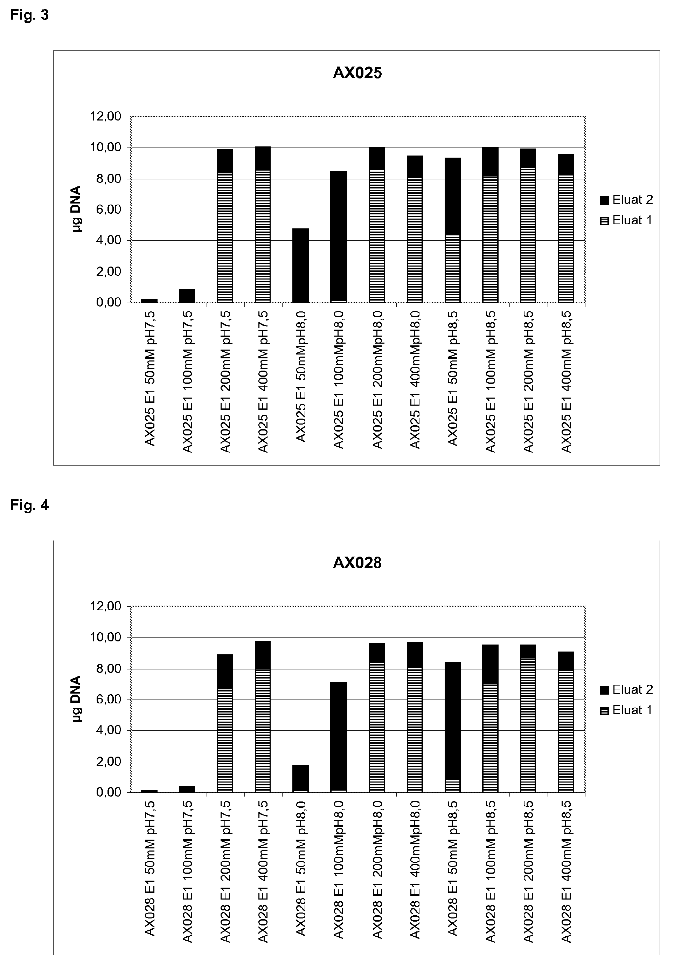

Die Ergebnisse sind in **FIG. 3** dargestellt.

### E) Aufreinigung von Plasmid pUC21 mit Pentaethylenhexamin funktionalisierten magnetischen Polymeren (AX 028)

Es werden 2 mg der magnetischen Partikel in 50 mM MES-Puffer, pH 6,1, eingesetzt und mit 50µl 50 mM Tris-Puffer, pH 7,0, versetzt. Dann setzt man 10µg Plasmid pUC21 in 10µl Puffer "EB" (QIAGEN, Katalog-Nr. 19068) zu und mischt durch kurzes Schütteln. Anschließend inkubiert man Reaktionsgemisch 5 lang am oder Eppendorf-Schüttler. Das Probengemisch wird magnetisch separiert und die Überstände entnommen und der Gehalt von DNA photometrisch bestimmt. Anschließend wäscht man die Rückstände zweimal mit je 100µl Millipore-Wasser, separiert magnetisch und verwirft die Überstände. Dann eluiert man zweimal durch Zugabe von je 50µl 50 mM Tris-Puffer, pH 7,5, 8,0 und 8,5 mit NaCl-Konzentrationen von jeweils 50 mM, 100 mM, 200 mM und 400 mM, trennt mittels magnetischer Separation ab untersucht die Eluate photometrisch auf deren DNA-Gehalt.

Die Ergebnisse sind in **FIG. 4** dargestellt.

### F) Aufreinigung von Plasmid pUC21 mit Polyallylamin funktionalisierten magnetischen Polymeren (AX 029) - Vergleichsbeispiel

Es werden 2 mg der magnetischen Partikel in 50 mM MES-Puffer, pH 6,1, eingesetzt und mit 50µl 50 mM Tris-Puffer, pH 7,0, versetzt. Dann setzt man 10µg Plasmid pUC21 in 10 µl Puffer "EB" (QIAGEN, Katalog-Nr. 19068) zu und mischt durch kurzes Schütteln. Anschließend inkubiert man das Reaktionsgemisch 5 Minuten lang am Überkopfschüttler oder Eppendorf-Schüttler. Das Probengemisch wird magnetisch separiert und die Überstände entnommen und der Gehalt von DNA photometrisch bestimmt. Anschließend wäscht man die Rückstände zweimal mit je 100µl Millipore-Wasser, separiert magnetisch und verwirft die Überstände. Dann eluiert man zweimal durch Zugabe von je 50 µl 50 mM Tris-Puffer, pH 7,5, 8,0 und 8,5 mit NaCl-Konzentrationen von jeweils 50 mM, 100 mM, 200 mM und 400 mM, trennt mittels magnetischer Separation ab und untersucht die Eluate photometrisch auf deren DNA-Gehalt.

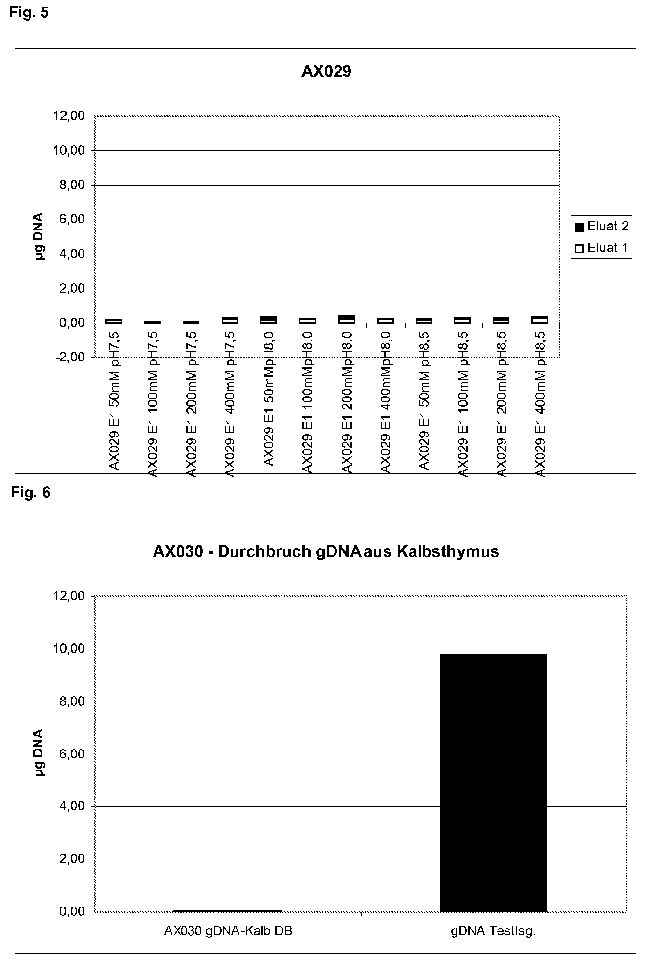

Die Ergebnisse sind in **FIG. 5** dargestellt.

### G) Aufreinigung von genomischer DNA mit Spermin funktionalisierten magnetischen Polymeren (AX 030)

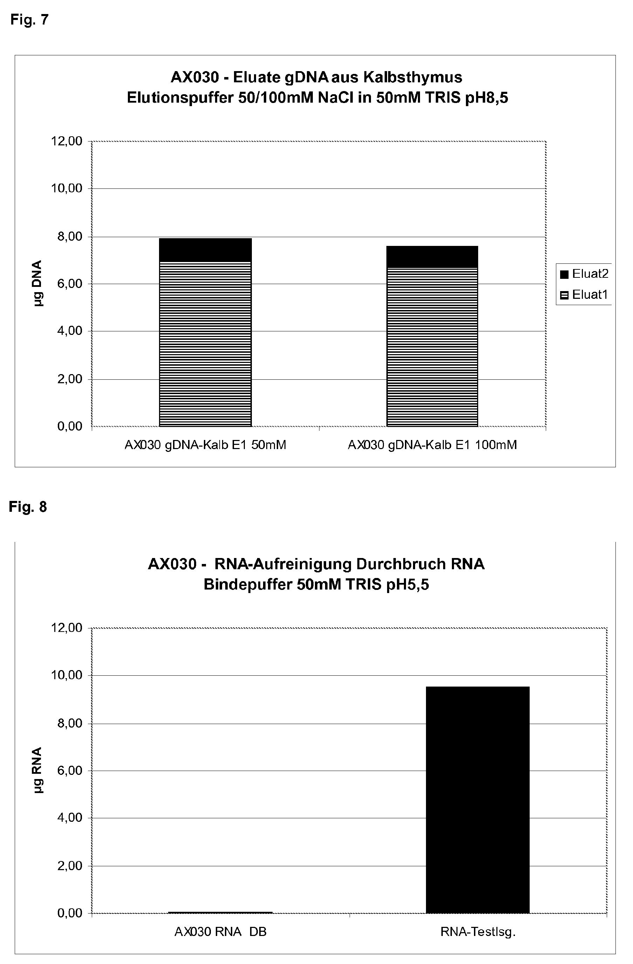

Man suspendiert pro Aufreinigung 2 mg Partikel in 25 mM MES, 25 mM Tris, pH 6,2. Dann setzt man 10 µg genomische DNA aus Kalbsthymus (Katalog-Nr. 89370, Fluka) in Puffer "EB" zu und mischt durch kurzes Schütteln. Anschließend separiert man magnetisch und untersucht die photometrisch. Man wäscht den Rückstand 100µl Millipore-Wasser mit magnetischer Separation zur Abtrennung der Überstände und eluiert dann zweimal mit je 50 µl 50 mM TRIS Puffer und 50 mM bzw. 100 mM NaCl, pH 8,5. Dann wird der DNA-Gehalt der einzelnen Eluate photometrisch bestimmt.
Die Ergebnisse sind in **FIG. 6** und **7** dargestellt.

### H) Aufreinigung von RNA mit Spermin funktionalisierten magnetischen Polymeren (AX 030)

Man suspendiert pro Aufreinigung 2 mg Partikel in 50 mM Tris Puffer, pH 5,5. Dann werden 10 µg / prep. RNA (16S-& 23S ribosomal, Fermentas 4I-1g/I-II) in 50 mM Tris-Puffer, pH 5,5, zugegeben. Man mischt kurz durch Schütteln, separiert magnetisch und untersucht dann die Überstände photometrisch auf RNA. Anschließend wäscht man zweimal mit je 100 µl RNase-freiem Wasser und entfernt die Überstände durch magnetische Separation. Dann eluiert man zweimal mit je 50 µl 50 mM TRIS (RNase-frei), pH 8,5 und 50 mM bzw. 100 mM NaCl. Die Eluate werden dann separat photometrisch untersucht.

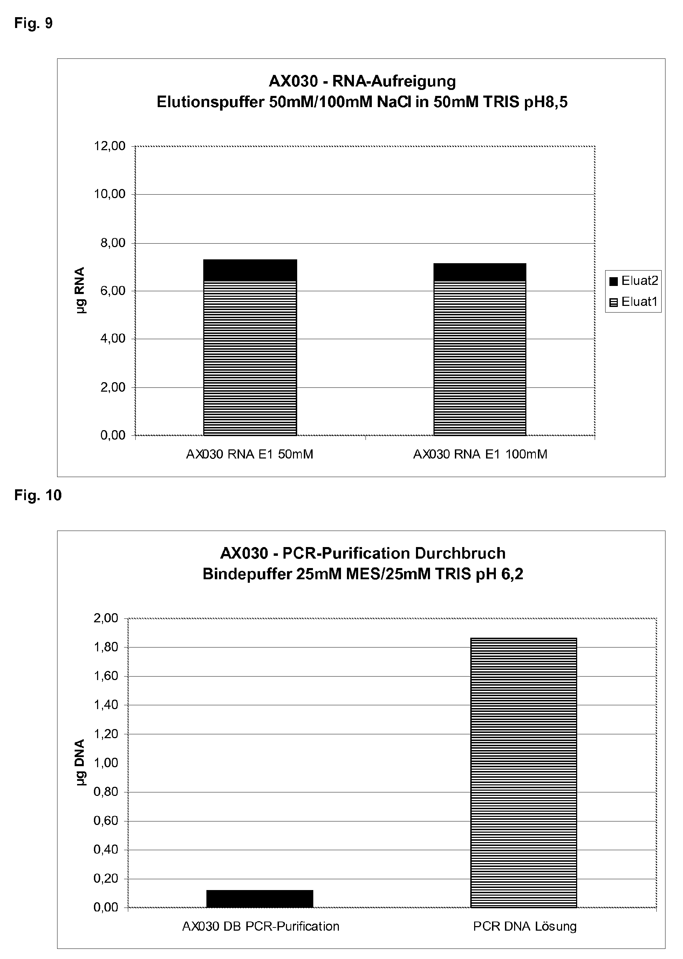

Die Ergebnisse sind in **FIG. 8** und **9** dargestellt.

### I) Aufreinigung von Nukleinsäurefragmenten mit Spermin funktionalisierten magnetischen Polymeren (AX 030)

### Vorbereitung

Die mit Spermin funktionalisierten magnetischen Polymerpartikel werden in einem Bindungspuffer mit 25 mM MES, 25 mM Tris, pH 6,2 mit einer Suspensionsdichte von 50 mg/ml suspendiert. Anschließend werden die Beads noch zweimal mit diesem Puffer gewaschen und die Überstände mittels magnetischer Separation abgetrennt. Man benötigt Plasmid DNA des Typs pTZ19R; von dem 25 µg DNA für 100 µl Pufferlösung eingesetzt werden sollen. Zunächst berechnet man sämtliche Mengen für den Ansatz, dann legt man die an 100 µl fehlende Menge Wasser vor, setzt die DNA zu, anschließend den zur Enzymlösung passenden Enzympuffer (1 µl pro 10 µl Gesamtlösung), dann zum Schluss pro µg DNA 3U Restriktionsenzym (Hinf I, New England Biolabs, Cat. # R0155S) zu (meist entsprechen 75 U 7,5 µl Enzymlösung) zu. Man lässt 90 Minuten bei 37°C im Wasserbad oder Heat-Block inkubieren. Dann zentrifugiert man kurz mit Quick-Run auf 6.000 U/min und friert anschließend die Proben bei -20°C ein. Diese durch Restriktion zerlegte DNA benutzt man als einfachen und schnellen PCR-Ersatz zum Test der PB Puffer.

### Durchführung

Man nimmt pro Probe 8µl der PCR-Lösung (entspricht 2µg DNA), füllt mit 92 µl 25 mM MES, 25 mM Tris, pH 6,2, auf, gibt 25 µl der magnetischen Kieselgelsuspensionen zu, vortext 10 Sekunden, versetzt mit 500µl 25 mM MES, 25 mM Tris, pH 6,2, und mixt. Man inkubiert in einem Schüttler 2 Minuten. Dann separiert man magnetisch, verwirft den Überstand und wäscht zweimal mit je 750 µl Millipore-Wasser. Anschließend eluiert man erst mit 50µl, dann mit 30 µl 50 mM Tris/HCl, 50 mM NaCl, pH 8,5. Man vereinigt die Eluate und untersucht sie photometrisch, sowie als Gel auf DNA Gehalt.

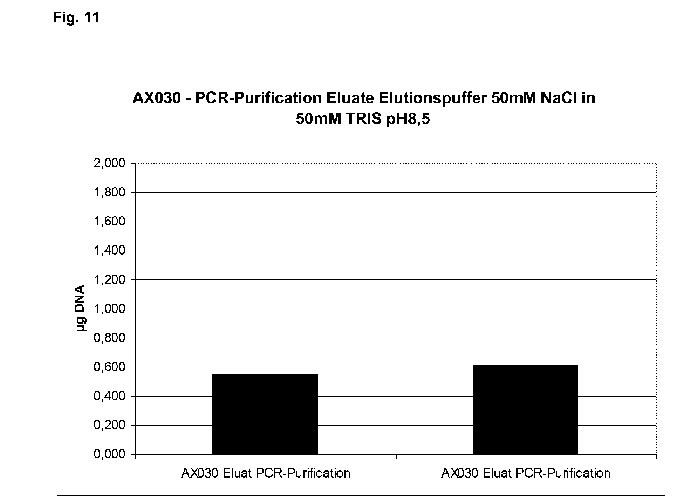

Die Ergebnisse sind in **FIG. 10****,** **11** und **12** dargestellt.

### J) Aufreinigung von genomischer DNA aus Blut mit Spermin funktionalisierten magnetischen Polymeren (AX 040)

1 ml Lysepuffer (10 mM TRIS, Triton X-100, pH 9,0) und 10 µl Proteinase K werden gemischt. 1 mg Beads und 3,4 µl Wasser werden in 200 µl Bindpuffer (1,5 M Kaliumacetat pH 4,0) mit einer Suspensionsdichte von 26,6 mg/ml suspendiert. 100 µl aufgetautes Vollblut (Citrat stabilisiert) werden in einem Eppendorfgefäß mit dem Lysepuffer/Proteinase-Mix versetzt und gut gemischt. Es wird bei Raumtemperatur 10 Minuten inkubiert. Die Beads werden im Bindepuffer sorgfältig resuspendiert. Davon werden 240 µl zu dem lysierten Blut gegeben. Es wird sorgfältig gemischt durch Auf- und Abpipettieren. Bei Raumtemperatur wird 1 Minute inkubiert. Die Beads werden magnetisch separiert. Der Überstand wird vorsichtig abgenommen. Zum Waschen wird 1 ml Waschpuffer (Wasser) zugeben. Es wird sorgfältig gemischt durch Auf- und Abpipettieren. Es wird erneut magnetisch separiert und der Überstand verworfen. Es werden 1 ml Lysepuffer und 50 µl Bindepuffer zugegeben, sorgfältig gemischt und eine Minute bei RT inkubiert. Es wird magnetisch separiert. Anschließend wird ein weiteres Mal mit 1 ml Waschpuffer gewaschen und magnetisch separiert.
Um die aufgereinigte DNA zu eluieren werden die Beads mit 150 µl Elutionspuffer (10 mM TRIS*HCl pH 8,5) versetzt. Die Beads werden durch mehrmaliges Auf- und Abpipettieren resuspendiert. Die Beads werden magnetisch separiert, der Überstand wird abgenommen und die DNA photometrisch quantifiziert.

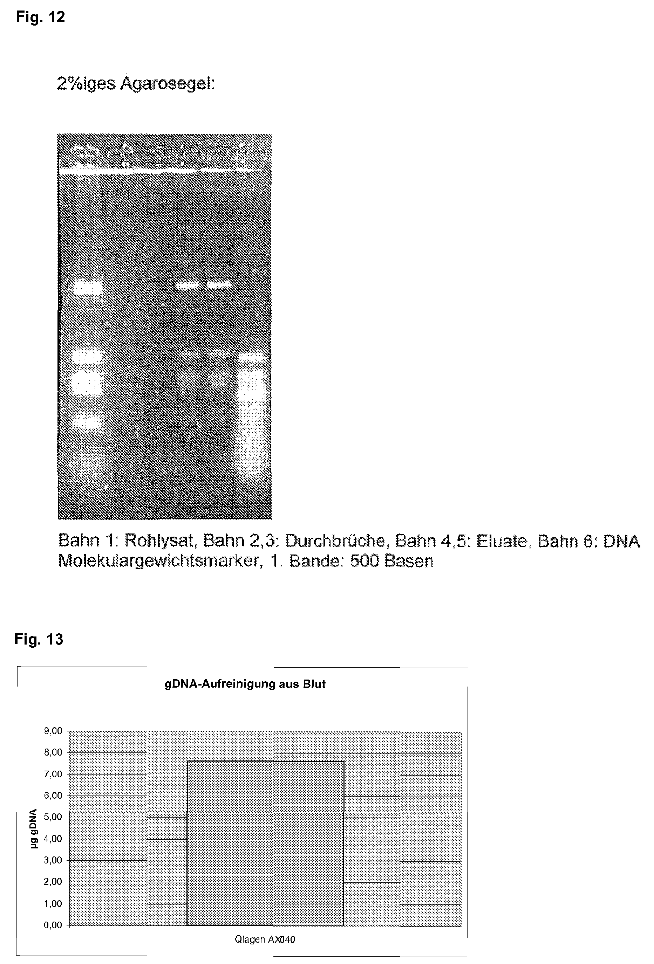

Die Ergebnisse sind in **FIG. 13** dargestellt.

5 µl des oben erhaltenen Eluates werden einer RT-PCR unterworfen. Dafür werden TaqMan β-actin Kontroll-Reagenz (Applied Biosystems, No. 401846) und QuantiTect Probe PCR Mastermix (QIAGEN, No. 1019337) verwendet, wobei 12,5 µl Mastermix, 2,5 µl β-actin Probe (FAM), 2,5 µl β-actin Forward Primer und 2,5 µl β-actin Reverse Primer eingesetzt werden.

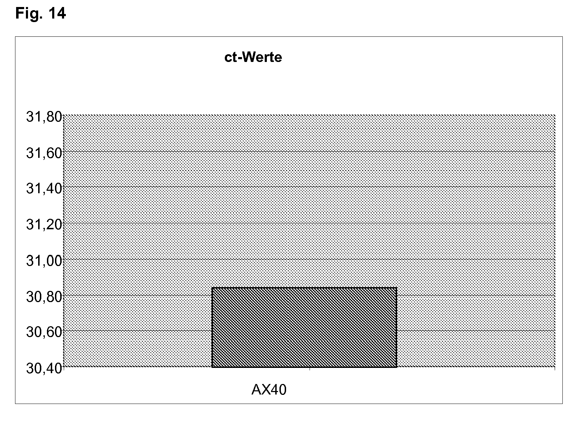

Die Ergebnisse sind in **FIG. 14** dargestellt.

## Patentansprüche

1. Verfahren zur Aufreinigung von DNA aus einer nukleinsäurehaltigen Probe, das wenigstens die nachfolgenden Schritte aufweist:
a. Inkontaktbringen der nukleinsäurehaltigen Probe mit einer nukleinsäurebindenden Phase, welche nukleinsäurebindende Gruppen aufweist, wobei die nukleinsäurebindenden Gruppen wenigstens eine protonierbare Gruppe aufweisen, die einen pKs-Wert von 9 bis 12 aufweist, wobei die nukleinsäurebindende Phase eine feste Phase ist und die nukleinsäurebindenden Gruppen kovalent an einen Träger gebunden vorliegen und wobei die nukleinsäurebindenden Gruppen ausgewählt sind aus der Gruppe der primären, sekundären und tertiären Mono- und Polyamine und wobei der Träger und/oder die nukleinsäurebindenden Gruppen Kationenaustauscher als funktionelle Gruppen aufweisen, die beim Elutions pH-Wert die Freisetzung der Nukleinsäuren fördern;
b. Bindung der DNA an die nukleinsäurebindende Phase bei einem pH-Wert (Bindungs pH-Wert), der wenigstens eine pH Einheit unterhalb des pKs-Wertes wenigstens einer der protonierbaren Gruppen liegt;
c. Elution der DNA bei einem pH-Wert, der oberhalb des Bindungs pH-Wertes liegt, jedoch wenigstens eine pH-Einheit unterhalb des pKs-Wertes wenigstens einer der protonierbaren Gruppen liegt und wobei die Elution bei einem pH-Wert von 8,2 bis 10 und bei einer Salzkonzentration von weniger als 0,25M erfolgt.

2. Das Verfahren nach Anspruch 1, **dadurch gekennzeichnet, dass** als Kationenaustauscher saure Gruppen vorliegen.

3. Das Verfahren nach Anspruch 2, **dadurch gekennzeichnet, dass** die sauren Gruppen Carboxylgruppen sind.

4. Das Verfahren nach einem der Ansprüche 1 bis 3, **dadurch gekennzeichnet, dass** der Träger mit einer Mischung aus nukleinsäurebindenden Gruppen und Verdünnungsgruppen beschichtet ist.

5. Das Verfahren nach einem oder mehreren der Ansprüche 1 bis 4, **dadurch gekennzeichnet, dass** die Bindung unter Bedingungen erfolgt, die wenigstens eines der nachfolgenden Merkmale aufweisen:
a. die Bindung erfolgt bei einem pH-Wert von 3 bis 8; und/oder
b. die Bindung erfolgt bei einem pH-Wert von 4 bis 7,5; und/oder
c. die Bindung erfolgt bei einem pH-Wert von 4,5 bis 7; und/oder
d. die Bindung erfolgt bei einem pH-Wert von 5,5 bis 7; und/oder
e. die Bindung erfolgt bei einem pH-Wert von 6,5 bis 7; und/oder
f. die Bindung erfolgt bei einer Salzkonzentration von weniger als 1 M, weniger als 0,5M, weniger als 0,25M oder weniger als 0,1 M.

6. Das Verfahren nach einem oder mehreren der Ansprüche 1 bis 5, **dadurch gekennzeichnet, dass** die Elution unter Bedingungen erfolgt, die wenigstens eines der nachfolgenden Merkmale aufweisen:
a. die Elution erfolgt bei einem pH-Wert von 8,2 bis 9; und/oder
b. die Elution erfolgt bei einem pH-Wert von 8,2 bis 8,8; und/oder
c. die Elution erfolgt bei einer Salzkonzentration von weniger als 0,1 M, weniger als 25mM, weniger als 15mM. oder weniger als 10mM; und/oder
d. zur Elution wird eine Lösung eingesetzt, die ausgewählt ist aus der Gruppe von Wasser, biologische und organische Puffer.

7. Das Verfahren nach einem oder mehreren der Ansprüche 1 bis 6, **dadurch gekennzeichnet, dass** nach der Bindung und vor der Elution ein Waschschritt durchgeführt wird.

8. Das Verfahren nach Anspruch 7, **dadurch gekennzeichnet, dass** eine Waschlösung eingesetzt wird, die eines oder mehrere der folgenden Merkmale aufweist:
a. für den Waschschritt wird Wasser oder eine wässrige Lösung mit niedriger Salzkonzentration eingesetzt; und/oder
b. es wird eine wässrige Lösung mit niedriger Salzkonzentration eingesetzt, wobei die Salzkonzentration weniger als 400mM, weniger als 200mM, weniger als 100mM, weniger als 50mM und/oder weniger als 25mM beträgt.

9. Das Verfahren nach einem oder mehreren der Ansprüche 1 bis 8, **dadurch gekennzeichnet, dass** die protonierbaren Gruppen eines oder mehrere der nachfolgenden Merkmale aufweisen:
a. die protonierbaren Gruppen sind Aminogruppen, die einen pKs-Wert von 10 bis 12 aufweisen; und/oder
b. die nukleinsäurebindenden Gruppen weisen pro Gruppe 1 bis 10, vorzugsweise 2 bis 8 und besonders bevorzugt 2 bis 6 Aminogruppen auf; und/oder
c. die nukleinsäurebindenden Gruppen sind ausgewählt aus Spermin und/oder Spermidin; und/oder
d. die protonierbaren Gruppen sind Aminogruppen und liegen nicht in Konjugation mit die Elektronendichte verringernden Gruppen vor.

10. Das Verfahren nach einem oder mehreren der Ansprüche 1 bis 9, **dadurch gekennzeichnet, dass** die nukleinsäurebindenden Gruppen ausgewählt sind aus der Gruppe der primären und sekundären Mono- und Polyamine.

11. Das Verfahren nach einem oder mehreren der Ansprüche 1 bis 10, **dadurch gekennzeichnet, dass** die nukleinsäurebindenden Gruppen ausgewählt sind aus der Gruppe von Aminen gemäß den Formeln:
R₁R₂R₃N,
R₁R₂N(CH₂)ₙNR₃R₄
R₁R₂N(CH₂)ₙNR₃(CH₂)ₘNR₄R₅
R₁R₂N(CH₂)ₙNR₃(CH₂)ₘNR₄(CH₂)ₒNR₅R₆
R₁R₂N(CH₂)ₙNR₃(CH₂)ₘNR₄(CH₂)ₒNR₅(CH₂)ₚNR₆R₇
R₁R₂N(CH₂)ₙNR₃(CH₂)ₘNR₄(CH₂)ₒNR₅(CH₂)ₚNR₆(CH₂)_{q}NR₇R₈
R₁R₂N(CH₂)ₙNR₃(CH₂)ₘNR₄(CH₂)ₒNR₅(CH₂)ₚNR₆(CH₂)_{q}NR₇(CH₂)ᵣNR₈R₉
R₁R₂N(CH₂)ₙNR₃(CH₂)ₘNR₄(CH₂)₀NR₅(CH₂)ₚNR₆(CH₂)_{q}NR₇(CH₂)ᵣNR₈(CH₂)ₛNR₉R₁₀
wobei
n, m, o, p, q, r und s unabhängig voneinander 2 bis 8 sind;
R₁, R₂, R₃, R₄, R₅, R₆, R₇, R₈, Rg und R₁₀ gleich oder verschieden sind und ausgewählt sind aus der Gruppe H, Alkyl (verzweigt oder unverzweigt) und Aryl.

12. Verfahren nach einem oder mehreren der Ansprüche 1 bis 11, **dadurch gekennzeichnet, dass** die nukleinsäurebindenden Gruppen ausgewählt sind aus N-Propyl-1,3-propandiamin, Pentaethylenhexamin, Spermin und Spermidin.

13. Verwendung einer nukleinsäurebindenden Phase, die nukleinsäurebindende Gruppen mit wenigstens einer protonierbaren Gruppe aufweist, wobei die protonierbare Gruppe einen pKs-Wert von 9 bis 12 aufweist und wobei die nukleinsäurebindende Phase eine feste Phase ist und die nukleinsäurebindenden Gruppen kovalent an einen Träger gebunden vorliegen und wobei die nukleinsäurebindenden Gruppen ausgewählt sind aus der Gruppe der primären, sekundären und tertiären Mono- und Polyamine und wobei die nukleinsäurebindende Phase zusätzlich Kationenaustauscher als funktionelle Gruppen aufweist, die bei dem Elutions pH-Wert die Freisetzung/Elution der Nukleinsäuren begünstigen, zur Aufreinigung von DNA, wobei ein Elutions pH-Wert eingestellt wird, der oberhalb des Bindungs pH-Wertes liegt, jedoch wenigstens eine pH Einheit unterhalb des pKs-Wertes wenigstens einer der protonierbaren Gruppen liegt und wobei die Elution bei einem pH-Wert von 8,2 bis 10 und bei einer Salzkonzentration von weniger als 0,25M erfolgt.

14. Verwendung nach Anspruch 13, **dadurch gekennzeichnet, dass** die nukleinsäurebindende Phase wenigstens eines der nachfolgenden Merkmale aufweist:
a) als Kationenaustauscher liegen Carboxylgruppen vor; und/oder
b) die nukleinsäurebindende Phase weist einen Träger auf, der ausgewählt ist aus der Gruppe bestehend aus organische Polymere wie Polystyrol und seine Derivate, Polyacrylate und -methacrylate und ihre Derivate, Polyurethane, Nylon, Polyethylen, Polypropylen, Polybutylen und Copolymere aus diesen Materialien, Polysaccharide und Hydrogele wie Agarose, Cellulose, Dextran, Sephadex, Sephacryl, Chitosan, anorganische Träger, insbesondere Kieselgele, Silikapartikel, Glas oder weitere Metall- und Halbmetalloxide, Boroxid, Träger mit Metalloberflächen, wie z. B. Gold und magnetische Partikel; und/oder
c) die nukleinsäurebindenden Gruppen der nukleinsäurebindende Phase weisen primäre oder sekundäre Amine auf; und/oder
d) die nukleinsäurebindenden Gruppen nach Merkmal c) sind Spermin und/oder Spermidin.

15. Ein Kit zur Aufreinigung von DNA, **dadurch gekennzeichnet, dass** es
a. eine nukleinsäurebindende Phase aufweist, wobei die nukleinsäurebindende Phase nukleinsäurebindende Gruppen mit wenigstens einer protonierbaren Gruppe aufweist, wobei die protonierbare Gruppe einen pKs-Wert von 9 bis 12 aufweist und die nukleinsäurebindenden Gruppen ausgewählt sind aus N-Propyl-1,3-propandiamin, Pentaethylenhexamin, Spermin und Spermidin und wobei die nukleinsäurebindende Phase eine feste Phase ist und die nukleinsäurebindenden Gruppen kovalent an einen Träger gebunden vorliegen und wobei die nukleinsäurebindende Phase zusätzlich Kationenaustauscher als funktionelle Gruppen aufweist, die bei dem Elutions pH-Wert die Freisetzung/Elution der Nukleinsäuren begünstigen;
b. einen Bindungspuffer mit einem pH-Wert, der wenigstens eine pH Einheit unterhalb des pKs-Wertes wenigstens einer der protonierbaren Gruppen der nukleinsäurebindenden Phase liegt und/oder einen Bindungspuffer, der die Einstellung eines solchen pH-Wertes in der Probe ermöglicht, aufweist; und
c. einen Elutionspuffer mit einem pH-Wert, der eine pH Einheit unterhalb des pKs-Wertes wenigstens einer der protonierbaren Gruppen der nukleinsäurebindenden Phase liegt und der einen pH-Wert von 8,2 bis 10 und eine Salzkonzentration von weniger als 0,25M aufweist.

16. Das Kit nach Anspruch 15, **dadurch gekennzeichnet, dass**
a. der Bindungspuffer wenigstens eines der folgenden Merkmale aufweist:
i. einen pH-Wert von 3 bis 8; und/oder
ii. einen pH-Wert von 4 bis 7,5; und/oder
iii. einen pH-Wert von 4,5 bis 7; und/oder
iv. einen pH-Wert von 5,5 bis 7; und/oder
v. einen pH-Wert von 6,5 bis 7; und/oder
vi. eine Salzkonzentration von weniger als 0,25M oder weniger als 0,1 M;
und/oder
b. der Elutionspuffer wenigstens eines der folgenden Merkmale aufweist:
i. einen pH-Wert von 8,2 bis 9; und/oder
ii. einen pH-Wert von 8,2 bis 8,8; und/oder
iii. eine Salzkonzentration von weniger als 0,1M, weniger als 25mM, weniger als 15mM oder weniger als 10mM; und/oder
iv. er ist ausgewählt aus der Gruppe bestehend aus Wasser, biologische und organische Puffer.

17. Das Kit nach Anspruch 15 oder 16, **dadurch gekennzeichnet, dass** die nukleinsäurebindende Phase wenigstens eines der nachfolgenden Merkmale aufweist:
a) als Kationenaustauscher liegen Carboxylgruppen vor; und/oder
b) die nukleinsäurebindende Phase weist einen Träger auf, der ausgewählt ist aus der Gruppe bestehend aus organische Polymere wie Polystyrol und seine Derivate, Polyacrylate und -methacrylate und ihre Derivate, Polyurethane, Nylon, Polyethylen, Polypropylen, Polybutylen und Copolymere aus diesen Materialien, Polysaccharide und Hydrogele wie Agarose, Cellulose, Dextran, Sephadex, Sephacryl, Chitosan, anorganische Träger, insbesondere Kieselgele, Silikapartikel, Glas oder weitere Metall- und Halbmetalloxide, Boroxid, Träger mit Metalloberflächen, wie z. B. Gold und magnetische Partikel; und/oder
c) die nukleinsäurebindenden Gruppen sind Spermin und/oder Spermidin.

18. Das Kit nach einem oder mehreren der Ansprüche 15 bis 17, **dadurch gekennzeichnet, dass** die nukleinsäurebindende Phase N-propyl-1,3-propandiamin als nukleinsäurebindende Gruppen aufweist.

## Claims

1. Method of purifying DNA from a nucleic acid-containing sample, comprising at least the following steps:
a. contacting the nucleic acid-containing sample with a nucleic acid-binding phase which comprises nucleic acid-binding groups, said nucleic acid-binding groups comprising at least one protonatable group having a pKa value of from 9 to 12, wherein the nucleic acid-binding phase is a solid phase and the nucleic acid-binding groups are covalently bound to a support and wherein the nucleic acid-binding groups are selected from the group consisting of primary, secondary and tertiary mono- and polyamines and wherein the support and/or the nucleic acid-binding groups comprise cation exchangers as functional groups which promote the release of the nucleic acids at the elution pH;
b. binding the DNA to the nucleic acid-binding phase at a pH value (binding pH) which is at least one pH unit below the pKa value of at least one of the protonatable groups;
c. eluting the DNA at a pH value which is above the binding pH but at least one pH unit below the pKa value of at least one of the protonatable groups and wherein the elution is carried out at a pH value of from 8.2 to 10 and at a salt concentration of less than 0.25 M.

2. Method according to claim 1, **characterized in that** acidic groups are present as cation exchanger.

3. Method according to claim 2, **characterized in that** said acidic groups are carboxyl groups.

4. Method according to one of claims 1 to 3, **characterized in that** the support is coated with a mixture of nucleic acid-binding groups and diluting groups.

5. Method according to one or more of claims 1 to 4, **characterized in that** binding is carried out under conditions which have at least one of the following features:
a. binding is carried out at a pH value of from 3 to 8; and/or
b. binding is carried out at a pH value of from 4 to 7.5; and/or
c. binding is carried out at a pH value of from 4.5 to 7; and/or
d. binding is carried out at a pH value of from 5.5 to 7; and/or
e. binding is carried out at a pH value of from 6.5 to 7; and/or
f. binding is carried out at a salt concentration of less than 1 M, less than 0.5 M, less than 0.25 M or less than 0.1 M.

6. Method according to one or more of claims 1 to 5, **characterized in that** elution is carried out under conditions which fulfill at least one of the following features:
a. elution is carried out at a pH value of from 8.2 to 9; and/or
b. elution is carried out at a pH value of from 8.2 to 8.8; and/or
c. elution is carried out at a salt concentration of less than 0.1 M, less than 25 mM, less than 15 mM or less than 10 mM; and/or
d. elution is carried out using a solution selected from the group consisting of water, biological and organic buffers.

7. Method according to one or more of claims 1 to 6, **characterized in that** a washing step is carried out after binding and prior to elution.

8. Method according to claim 7, **characterized in that** a washing solution is used which has one or more of the following features:
a. for the washing step water or an aqueous solution with a low salt concentration is used; and/or
b. an aqueous solution with a low salt concentration is used, wherein said salt concentration is less than 400 mM, less than 200 mM, less than 100 mM, less than 50 mM, and/or less than 25 mM.

9. Method according to one or more of claims 1 to 8, **characterized in that** the protonatable groups fulfill one or more of the following features:
a. the protonatable groups are amino groups which have a pKa value of from 10 to 12; and/or
b. the nucleic acid-binding groups comprise per group from 1 to 10, preferably 2 to 8 and particularly preferred 2 to 6 amino groups; and/or
c. the nucleic acid-binding groups are selected from spermine and/or spermidine; and/or
d. the protonatable groups are amino groups and are not conjugated to electron density-reducing groups.

10. Method according to one or more of claims 1 to 9, **characterized in that** the nucleic acid-binding groups are selected from the group consisting of primary and secondary mono- and polyamines.

11. Method according to one or more of claims 1 to 10, **characterized in that** the nucleic acid-binding groups are selected from the group consisting of amines of the formulae:
R₁R₂R₃N,
R₁R₂N(CH₂)ₙNR₃R₄
R₁R₂N(CH₂)ₙNR₃(CH₂)ₘNR₄R₅
R₁R₂N(CH₂)ₙNR₃(CH₂)ₘNR₄(CH₂)ₒNR₅R₆
R₁R₂N(CH₂)ₙNR₃(CH₂)ₘNR₄(CH₂)ₒNR₅(CH₂)ₚNR₆R₇
R₁R₂N(CH₂)ₙNR₃(CH₂)ₘNR₄(CH₂)ₒNR₅(CH₂)ₚNR₆(CH₂)_{q}NR₇R₈
R₁R₂N(CH₂)ₙNR₃(CH₂)ₘNR₄(CH₂)₀NR₅(CH₂)pNR₆(CH₂)qNR₇(CH₂)ᵣNR₈R₉
R₁R₂N(CH₂)ₙNR₃(CH₂)ₘNR₄(CH₂)ₒNR₅(CH₂)NR₆(CH₂)_{q}NR₇(CH₂)ᵣNR₈(CH₂)NR₉R₁₀
wherein
n, m, o, p, q, r and s are, independently of another, 2 to 8;
R₁, R₂, R₃, R₄, R₅, R₆, R₇, R₈, Rg und R₁₀ are identical or different and are selected from the group consisting of H, alkyl (branched or unbranched) and aryl.

12. Method according to one or more of claims 1 to 11, **characterized in that** the nucleic acid-binding groups are selected from N-propyl-1,3-propanediamine, pentaethylenehexamine, spermine and spermidine.

13. Use of a nucleic acid-binding phase which comprises nucleic acid-binding groups with at least one protonatable group, wherein the protonatable group has a pKa value of from 9 to 12 and wherein the nucleic acid-binding phase is a solid phase and the nucleic acid-binding groups are bound covalently to a support and wherein the nucleic acid-binding groups are selected from the group consisting of primary, secondary and tertiary mono- and polyamines and wherein the nucleic acid-binding phase comprises additionally cation exchangers as functional groups which promote the release/elution of the nucleic acids at the elution pH value, for purifying DNA, wherein an elution pH is used which is above the binding pH but at least one pH unit below the pKa value of at least one of the protonatable groups and wherein the elution is carried out at a pH value of from 8.2 to 10 and at a salt concentration of less than 0.25 M.

14. Use according to claim 13, **characterized in that** the nucleic acid-binding phase has at least one of the following features:
a) carboxyl groups are present as cation exchangers; and/or
b) the nucleic acid-binding phase comprises a support which is selected from the group consisting of organic polymers such as polystyrene and its derivatives, polyacrylates and polymethacrylates and their derivatives, polyurethanes, nylon, polyethylene, polypropylene, polybutylene and copolymers of these materials, polysaccharides and hydrogels such as agarose, cellulose, dextran, Sephadex, Sephacryl, chitosan, inorganic supports, in particular silica gels, silica particles, glass or other metal and semi-metal oxides, boron oxide, supports with metal surfaces, for example gold and magnetic particles; and/or
c) the nucleic acid-binding groups of the nucleic acid-binding phase comprise primary or secondary amines; and/or
d) the nucleic acid-binding groups according to feature c) are spermine and/or spermidine.

15. Kit for purifying DNA, **characterized in that** it
a. comprises a nucleic acid-binding phase, wherein the nucleic acid-binding phase comprises nucleic acid-binding groups with at least one protonatable group, wherein the protonatable group has a pKa value of from 9 to 12 and wherein the nucleic acid-binding groups are selected from N-propyl-1,3-propanediamine, pentaethylenehexamine, spermine and spermidine and wherein the nucleic acid-binding phase is a solid phase and the nucleic acid-binding groups are bound covalently to a support and wherein the nucleic acid-binding phase additionally comprises cation exchangers as functional groups which promote the release/elution of the nucleic acids at the elution pH;
b. comprises a binding buffer with a pH value which is at least one pH unit below the pKa value of at least one of the protonatable groups of the nucleic acid-binding phase, and/or a binding buffer which enables the adjustment of such a pH value in the sample; and
c. comprises an elution buffer with a pH value which is one pH unit below the pKa value of at least one of the protonatable groups of the nucleic acid-binding phase and which has a pH value of from 8.2 to 10 and a salt concentration of less than 0.25 M.

16. Kit according to claim 15, **characterized in that**
a. the binding buffer has at least one of the following features:
i. a pH value of from 3 to 8; and/or
ii. a pH value of from 4 to 7.5; and/or
iii. a pH value of from 4.5 to 7; and/or
iv. a pH value of from 5.5 to 7; and/or
v. a pH value of from 6.5 to 7; and/or
vi. a salt concentration of less than 0.25 M or less than 0.1 M;
and/or
b. the elution buffer has at least one of the following features:
i. a pH value of from 8.2 to 9; and/or
ii. a pH value of from 8.2 to 8.8; and/or
iii. a salt concentration of less than 0.1 M, less than 25 mM, less than 15 mM, or less than 10 mM; and/or
iv. it is selected from the group consisting of water, biological an organic buffers.

17. Kit according to claim 15 or 16, **characterized in that** the nucleic acid-binding phase hast at least one of the following features:
a) carboxyl groups are present as cation exchanger; and/or
b) the nucleic acid-binding phase comprises a support which is selected from the group consisting of organic polymers such as polystyrene and its derivatives, polyacrylates and polymethacrylates and their derivatives, polyurethanes, nylon, polyethylene, polypropylene, polybutylene and copolymers of these materials, polysaccharides and hydrogels such as agarose, cellulose, dextran, Sephadex, Sephacryl, chitosan, inorganic supports, in particular silica gels, silica particles, glass or other metal and semi-metal oxides, boron oxide, supports with metal surfaces, such as for example gold and magnetic particles; and/or
c) the nucleic acid-binding groups are spermine and/or spermidine.

18. Kit according to one or more of claims 15 to 17, **characterized in that** the nucleic acid-binding phase comprises N-propyl-1,3-propanediamine as nucleic acid-binding groups.

## Revendications

1. Procédé de purification d'ADN d'un échantillon contenant des acides nucléiques, qui présente au moins les étapes suivantes :
a. mise en contact de l'échantillon contenant des acides nucléiques avec une phase liant les acides nucléiques qui présente des groupes liant les acides nucléiques, les groupes liant les acides nucléiques présentant au moins un groupe protonable, qui présente une valeur de pKa de 9 à 12, la phase liant les acides nucléiques étant une phase solide et les groupes liant les acides nucléiques se trouvant sous forme liée par covalence à un support et les groupes liant les acides nucléiques étant choisis dans le groupe des monoamines et des polyamines primaires, secondaires et tertiaires et le support et/ou les groupes liant les acides nucléiques présentant des échangeurs de cations comme groupes fonctionnels, qui favorisent, au pH de l'élution, la libération des acides nucléiques;
b. liaison de l'ADN à la phase liant les acides nucléiques à un pH (pH de liaison) qui se situe à au moins une unité de pH sous la valeur de pKa d'au moins un des groupes protonables;
c. élution de l'ADN à un pH, qui est supérieur au pH de liaison mais qui se situe cependant au moins une unité de pH sous la valeur de pKa d'au moins un des groupes protonables et l'élution ayant lieu à un pH de 8,2 à 10 et à une concentration en sel inférieure à 0,25 M.

2. Procédé selon la revendication 1, **caractérisé en ce qu'**il s'agit, pour les échangeurs de cations, de groupes acides.

3. Procédé selon la revendication 2, **caractérisé en ce que** les groupes acides sont des groupes carboxyle.

4. Procédé selon l'une quelconque des revendications 1 à 3, **caractérisé en ce que** le support est revêtu d'un mélange de groupes liant les acides nucléiques et de groupes de dilution.

5. Procédé selon l'une ou plusieurs des revendications 1 à 4, **caractérisé en ce que** la liaison a lieu dans des conditions qui présentent au moins une des caractéristiques suivantes :
a. la liaison a lieu à un pH de 3 à 8; et/ou
b. la liaison a lieu à un pH de 4 à 7,5; et/ou
c. la liaison a lieu à un pH de 4,5 à 7; et/ou
d. la liaison a lieu à un pH de 5,5 à 7; et/ou
e. la liaison a lieu à un pH de 6,5 à 7; et/ou
f. la liaison a lieu à une concentration en sel inférieure à 1 M, inférieure à 0,5 M, inférieure à 0,25 M ou inférieure à 0,1 M.

6. Procédé selon l'une ou plusieurs des revendications 1 à 5, **caractérisé en ce que** l'élution a lieu dans des conditions qui présentent au moins une des caractéristiques suivantes :
a. l'élution a lieu à un pH de 8,2 à 9; et/ou
b. l'élution a lieu à un pH de 8,2 à 8,8; et/ou
c. l'élution a lieu à une concentration en sel inférieure à 0,1 M, inférieure à 25 mM, inférieure à 15 mM ou inférieure à 10 mM; et/ou
d. on utilise, pour l'élution, une solution qui est choisie dans le groupe formé par l'eau, les tampons biologiques et organiques.

7. Procédé selon l'une ou plusieurs des revendications 1 à 6, **caractérisé en ce qu'**une étape de lavage est réalisée après la liaison et avant l'élution.

8. Procédé selon la revendication 7, **caractérisé en ce qu'**on utilise une solution de lavage qui présente une ou plusieurs des caractéristiques suivantes:
a. pour l'étape de lavage, on utilise de l'eau ou une solution aqueuse présentant une faible concentration en sel; et/ou
b. on utilise une solution aqueuse présentant une faible concentration en sel, la concentration en sel étant inférieure à 400 mM, inférieure à 200 mM, inférieure à 100 mM, inférieure à 50 mM et/ou inférieure à 25 mM.

9. Procédé selon l'une ou plusieurs des revendications 1 à 8, **caractérisé en ce que** les groupes protonables présentent une ou plusieurs des caractéristiques suivantes:
a. les groupes protonables sont des groupes amino qui présentent une valeur de pKa de 10 à 12; et/ou
b. les groupes liant les acides nucléiques présentent, par groupe, 1 à 10, de préférence 2 à 8 et de manière particulièrement préférée 2 à 6 groupes amino; et/ou
c. les groupes liant les acides nucléiques sont choisis parmi la spermine et/ou la spermidine; et/ou
d. les groupes protonables sont des groupes amino et ne se trouvent pas sous forme conjuguée avec les groupes diminuant la densité électronique.

10. Procédé selon l'une ou plusieurs des revendications 1 à 9, **caractérisé en ce que** les groupes liant les acides nucléiques sont choisis dans le groupe des monoamines et des polyamines primaires et secondaires.

11. Procédé selon l'une ou plusieurs des revendications 1 à 10, **caractérisé en ce que** les groupes liant les acides nucléiques sont choisis dans le groupe des amines selon les formules:
R₁R₂R₃N,
R₁R₂N(CH₂)ₙNR₃R₄
R₁R₂N(CH₂)ₙNR₃(CH₂)ₘNR₄R₅
R₁R₂N(CH₂)ₙNR₃(CH₂)ₘNR₄(CH₂)ₒNR₅R₆
R₁R₂N(CH₂)ₙNR₃(CH₂)ₘNR₄(CH₂)_{c}NR₅(CH₂)ₚNR₆R₇
R₁R₂N(CH₂)NR₃(CH₂)ₘNR₄(CH₂)ₒNR₅(CH₂)ₚNR₆(CH₂)_{q}NR₇R₈
R₁R₂N(CH₂)ₙNR₃(CH₂)ₘNR₄(CH₂)ₒNR₅(CH₂)ₚNR₆(CH₂)_{q}NR₇(CH₂)ᵣNR₈R₉
R₁R₂N(CH₂)ₙNR₃(CH₂)ₘNR₄(CH₂)ₒNR₅(CH₂)ₚNR₆(CH₂)_{q}NR₇(CH₂)ᵣNR₈(CH₂)ₛNR₉R₁₀
où
n, m, o, p, q, r et s valent, indépendamment les uns des autres, 2 à 8,
R₁, R₂, R₃, R₄, R₅, R₆, R₇, R₈, Rg et R₁₀ sont identiques ou différents et sont choisis dans le groupe formé par H, alkyle (linéaire ou ramifié) et aryle.

12. Procédé selon l'une ou plusieurs des revendications 1 à 11, **caractérisé en ce que** les groupes liant les acides nucléiques sont choisis parmi la N-propyl-1,3-propanediamine, la pentaéthylènehexamine, la spermine et la spermidine.

13. Utilisation d'une phase liant les acides nucléiques qui présente des groupes liant les acides nucléiques présentant au moins un groupe protonable, le groupe protonable présentant une valeur de pKa de 9 à 12 et la phase liant les acides nucléiques étant une phase solide et les groupes liant les acides nucléiques se trouvant sous forme liée par covalence à un support et les groupes liant les acides nucléiques étant choisis dans le groupe des monoamines et des polyamines primaires, secondaires et tertiaires et la phase liant les acides nucléiques présentant en outre des échangeurs de cations comme groupes fonctionnels, qui favorisent, au pH de l'élution, la libération/l'élution des acides nucléiques, pour la purification d'ADN, un pH d'élution étant réglé qui est situé au-dessus du pH de liaison mais cependant au moins une unité de pH sous la valeur de pKa d'au moins un des groupes protonables et l'élution ayant lieu à un pH de 8,2 à 10 et à une concentration en sel inférieure à 0,25 M.

14. Utilisation selon la revendication 13, **caractérisée en ce que** la phase liant les acides nucléiques présente au moins une des caractéristiques suivantes:
a) des groupes carboxyle sont présents en tant qu'échangeurs de cations; et/ou
b) la phase liant les acides nucléiques présente un support qui est choisi dans le groupe constitué par les polymères organiques, tels que le polystyrène et ses dérivés, les polyacrylates et les polyméthacrylates et leurs dérivés, les polyuréthanes, le Nylon, le polyéthylène, le polypropylène, le polybutylène et les copolymères de ces matériaux, les polysaccharides et les hydrogels, tels que l'agarose, la cellulose, le dextrane, le Sephadex, le Sephacryle, le chitosane, les supports inorganiques, en particulier les gels siliciques, les particules de silice, le verre ou d'autres oxydes métalliques ou semi-métalliques, l'oxyde de bore, les supports présentant des surfaces métalliques, telles que par exemple de l'or et des particules magnétiques; et/ou
c) les groupes liant les acides nucléiques de la phase liant les acides nucléiques présentent des amines primaires ou secondaires; et/ou
d) les groupes liant les acides nucléiques selon la caractéristique c) sont la spermine et/ou la spermidine.

15. Kit ou nécessaire pour la purification d'ADN, **caractérisé en ce qu'**il présente
a. une phase liant les acides nucléiques, la phase liant les acides nucléiques présentant des groupes liant les acides nucléiques présentant au moins un groupe protonable, le groupe protonable présentant une valeur de pKa de 9 à 12 et les groupes liant les acides nucléiques étant choisis parmi la N-propyl-1,3-propanediamine, la pentaéthylènehexamine, la spermine et la spermidine et la phase liant les acides nucléiques étant une phase solide et les groupes liant les acides nucléiques se trouvant sous forme liée par covalence à un support et la phase liant les acides nucléiques présentant en outre des échangeurs de cations comme groupes fonctionnels, qui favorisent, au pH de l'élution, la libération/l'élution des acides nucléiques;
b. un tampon de liaison présentant un pH qui est situé au moins une unité de pH sous la valeur de pKa d'au moins un des groupes protonables de la phase liant les acides nucléiques et/ou un tampon de liaison qui permet le réglage d'un tel pH dans l'échantillon; et
c. un tampon d'élution présentant un pH qui se situe une unité de pH sous la valeur de pKa d'au moins un des groupes protonables de la phase liant les acides nucléiques et qui présente un pH de 8,2 à 10 et une concentration en sel inférieure à 0,25 M.

16. Kit ou nécessaire selon la revendication 15, **caractérisé en ce que**
a. le tampon de liaison présente au moins une des caractéristiques suivantes:
i. un pH de 3 à 8; et/ou
ii. un pH de 4 à 7,5; et/ou
iii. un pH de 4,5 à 7; et/ou
iv. un pH de 5,5 à 7; et/ou
v. un pH de 6,5 à 7; et/ou
vi. une concentration en sel inférieure à 0,25 M ou inférieure à 0,1 M;
et/ou
b. le tampon d'élution présente au moins une des caractéristiques suivantes:
i. un pH de 8,2 à 9; et/ou
ii. un pH de 8,2 à 8,8; et/ou
iii. une concentration en sel inférieure à 0,1 M, inférieure à 25 mM, inférieure à 15 mM ou inférieure à 10 mM; et/ou
iv. il est choisi dans le groupe constitué par l'eau, les tampons biologiques et organiques.

17. Kit ou nécessaire selon la revendication 15 ou 16, **caractérisé en ce que** la phase liant les acides nucléiques présente au moins une des caractéristiques suivantes:
a) des groupes carboxyle sont présents en tant qu'échangeurs de cations; et/ou
b) la phase liant les acides nucléiques présente un support qui est choisi dans le groupe constitué par les polymères organiques, tels que le polystyrène et ses dérivés, les polyacrylates et les polyméthacrylates et leurs dérivés, les polyuréthanes, le Nylon, le polyéthylène, le polypropylène, le polybutylène et les copolymères de ces matériaux, les polysaccharides et les hydrogels, tels que l'agarose, la cellulose, le dextrane, le Sephadex, le Sephacryle, le chitosane, les supports inorganiques, en particulier les gels siliciques, les particules de silice, le verre ou d'autres oxydes métalliques ou semi-métalliques, l'oxyde de bore, les supports présentant des surfaces métalliques, telles que par exemple de l'or et des particules magnétiques; et/ou
c) les groupes liant les acides nucléiques sont la spermine et/ou la spermidine.

18. Kit ou nécessaire selon l'une ou plusieurs des revendications 15 à 17, **caractérisé en ce que** la phase liant les acides nucléiques présente de la N-propyl-1,3-propanediamine comme groupes liant les acides nucléiques.
